# EUROPÄISCHE PATENTSCHRIFT

(11) **EP 4 642 391 B1**
(45) Veröffentlichungstag und Bekanntmachung des Hinweises auf die Patenterteilung: **15.07.2026**
(21) Anmeldenummer: 23833750.5
(22) Anmeldetag: 18.12.2023
(51) Int. Cl.: A61F 2/66, A61F 2/70, A61F 2/72, A61F 2/76

(54) **ORTHOPÄDIETECHNISCHE GELENKEINRICHTUNG UND VERFAHREN ZU DEREN HERSTELLUNG**
ORTHOPAEDIC JOINT DEVICE, AND METHOD FOR PRODUCING SAME
DISPOSITIF ORTHOPÉDIQUE ET SON PROCÉDÉ DE FABRICATION

(30) Priorität: 28.12.2022 DE 102022134918
(43) Veröffentlichungstag der Anmeldung: 05.11.2025
(73) Patentinhaber: Otto Bock Healthcare Products GmbH, 1110 Wien (AT)
(72) Erfinder: SEIFERT, Dirk, 1110 Wien (AT)
(74) Vertreter: Gramm, Lins & Partner Patent- und Rechtsanwälte PartGmbB
(86) Internationale Anmeldenummer: PCT/EP2023/086364
(87) Internationale Veröffentlichungsnummer: WO 2024/141306

(56) Entgegenhaltungen:
- EP-B1- 3 285 694
- WO-A1-2021/124060

## Beschreibung

Die Erfindung betrifft ein Verfahren gemäß Anspruch 1.

Die Erfindung betrifft ebenfalls eine orthopädietechnische Gelenkeinrichtung gemäß Anspruch 15.

Die orthopädietechnische Gelenkeinrichtung ist insbesondere als Prothese, Orthese oder Exoskelett der oberen oder unteren Extremität ausgebildet, insbesondere für künstliche Knöchelgelenke, künstliche Kniegelenke, künstliche Hüftgelenke, künstliche Schultergelenke oder künstliches Ellenbogengelenk.

Orthopädietechnische Gelenkeinrichtungen wie Prothesen, Orthesen oder auch Exoskelette ermöglichen eine Verschwenkung zumindest zweier Komponenten um eine Schwenkachse, sodass ein Oberteil relativ zu einem Unterteil eine Schwenkbewegung um diese Schwenkachse ausführen kann. Bei einer Prothese kann das Oberteil des Gelenkes mit einem Prothesenschaft oder einem Ende-Exo-Implantat gekoppelt sein, um ein Unterteil und gegebenenfalls weitere prothetische Komponenten an einem Gliedmaßenstumpf festzulegen. Bei der Prothese einer unteren Extremität ist der Schaft beispielsweise als ein Oberschenkelschaft ausgebildet, der mit einem Oberteil eines Prothesenkniegelenkes gekoppelt ist und an dem ein Unterschenkelteil mit einem Prothesenfuß verschwenkbar gelagert sind. Zwischen dem Oberteil und dem Unterteil ist ein Aktuator angeordnet, mit dem die Relativverschwenkbewegung beeinflusst werden kann. Bei passiven Aktuatoren wird die Verschwenkbewegung beeinflusst oder verändert, indem ein Widerstand der Verschwenkbewegung entgegengesetzt wird. Der Widerstand ist veränderbar, um ein angepasstes Verschwenkverhalten erzeugen zu können. Die Anpassung erfolgt beispielsweise auf der Grundlage von Sensordaten, durch bestimmte Belastungen, Beschleunigungen oder Bewegungen oder auch Zustände. Passive Aktuatoren sind insbesondere pneumatische oder hydraulische Widerstände, magnetorheologische Widerstände oder andere Bremseinrichtungen, über die kinetische Energie in Wärmeenergie umgewandelt wird. Darüber hinaus gibt es aktive Aktuatoren, mit denen ebenfalls die Verschwenkbewegung von dem Oberteil relativ zu dem Unterteil beeinflusst werden kann. Antriebe, beispielsweise Motoren oder Kraftspeicher, können im Generatorbetrieb oder Ladebetrieb als Bremse wirken. Ebenso ist es möglich, eine Verschwenkbewegung über einen aktiven Aktuator dahingehend zu beeinflussen, dass aus einer Ruhestellung eine Verschwenkung stattfindet oder eine Verschwenkbewegung unterstützt wird. Durch die Aktivierung des Aktuators kann auch einer Verschwenkbewegung entgegengewirkt werden, ohne dass diese umgekehrt wird. Ein aktiver Aktuator wirkt dann ebenfalls als Bremse und moduliert die Verschwenkbewegung.

Moderne Steuerungen sehen die Beeinflussung der Verschwenkbewegung über den Aktuator auf der Grundlage von Sensorwerten vor, weil die Sensoren Zustandsdaten oder Bewegungsdaten der Gelenkeinrichtung erfassen. Auf der Grundlage von Sensorwerten wird der Aktuator aktiviert, deaktiviert oder moduliert, beispielsweise um nach Erreichen einer bestimmten Stellung, eines bestimmten Gelenkwinkels oder bei einer bestimmten Belastung einen entsprechenden Widerstand bereitzustellen oder eine Verschwenkung einzuleiten oder zu unterstützen. Darüber hinaus werden Aktuatoren aufgrund von Biosignalen aktiviert, deaktiviert oder moduliert. Biosignale sind Signale, die über zumindest einen Biosignalsensor erfasst werden und der Steuerungseinrichtung übermittelt werden. Biosignale sind insbesondere Signale, die eine Muskelaktivität, die Ansteuerung einer Muskulatur oder die Intention der Ansteuerung erkennen oder repräsentieren, beispielsweise durch eine mechanische Erfassung von Veränderungen mittels einer Druckmanschette oder dergleichen, durch elektromagnetische Sensoren, die myoelektrische Signale oder Nervensignale erfassen, oder durch elektromechanische Sensoren, die über Ultraschall oder Längenänderungen die Aktivitäten eines Patienten oder Nutzers der orthopädietechnischen Gelenkeinrichtung erfassen. Die Sensoren zur direkten oder indirekten Erfassung von Muskelaktivitäten oder der Aktivierung der Muskulatur sind die Biosignalsensoren. Auf der Grundlage der Biosignale, die der Steuerungseinrichtung übermittelt werden, wird der Aktuator aktiviert, deaktiviert oder moduliert, um die Verschwenkbewegung zu beeinflussen.

Die WO 2021/124060 A1 betrifft unter anderem ein Verfahren zum Steuern einer Prothese der oberen Extremitäten, die über EMG-Daten auf der Grundlage von Muskelkontraktionen gesteuert wird. Darüber hinaus werden Daten einer IMU oder Magnetfelddaten erfasst. Das Steuerungssignal zum Steuern der Protheseneinrichtung wird auf der Grundlage der EMG Daten und zumindest Daten eines der beiden anderen genannten Sensortypen erzeugt.

Die EP 3 285 694 B1 betrifft ein System zur Steuerung einer prothetischen oder orthetischen Vorrichtung mit einem ersten Teil und einem zweiten Teil, die über ein Gelenk miteinander verbunden sind. Das Gelenk weist eine neutrale Position auf, die während eines Gangzyklus verwendet wird, um einen Gelenkwinkel zwischen den beiden Teilen einzustellen. Ein erster Sensor ist als EMG-Sensor ausgebildet und übermittelt Signale von einem ersten Muskel an eine Steuerung. Ein Aktuator ist mit der Steuerung verbunden und verändert die neutrale Position. Weitere EMG-Sensoren sowie implantierte Sensoren oder Beschleunigungssensoren, Gyroskope, Magnetometer, Drucksensoren, Winkelsensoren oder dergleichen sind mit der Steuerung verbunden und verändern das Maß an Unterstützung durch den Aktuator.

Bestimmte Beeinflussungen der orthopädietechnischen Gelenkeinrichtung auf der Grundlage von Sensordaten erfordern von dem Nutzer der orthopädietechnischen Gelenkeinrichtung, dass bestimmte Zustände oder Positionen über einen längeren Zeitraum beibehalten werden. Soll eine Gelenkeinrichtung in einer bestimmten Position gesperrt werden, beispielsweise um eine Stehfunktion oder eine Haltefunktion auszuüben, muss die entsprechende orthopädietechnische Einrichtung für einen bestimmten Zeitraum in einer bestimmten Stellung bewegungslos oder nahezu bewegungslos gehalten werden. Die Steuerung erkennt dann, dass keine weitere Bewegung zu erwarten ist und verriegelt dann die Gelenkeinrichtung. Dies kann aufwendig und schwierig sein.

Aufgabe der vorliegenden Erfindung ist es, eine orthopädietechnische Gelenkeinrichtung und ein Verfahren zu deren Steuerung bereitzustellen, mit denen die Nutzung der orthopädietechnischen Gelenkeinrichtung für den Patienten erleichtert und sicherer gestaltet werden kann.

Erfindungsgemäß wird diese Aufgabe durch ein Verfahren mit den Merkmalen des Hauptanspruches und eine orthopädietechnische Gelenkeinrichtung mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das Verfahren zur Steuerung einer orthopädietechnischen Gelenkeinrichtung mit einem Oberteil und einem Unterteil, die gelenkig aneinander, um eine Schwenkachse verschwenkbar gelagert sind, mit einem Aktuator, der mit dem Oberteil und dem Unterteil gekoppelt ist und eine Verschwenkbewegung des Oberteils relativ zu dem Unterteil beeinflusst, wobei der Aktuator mit einer Steuerungseinrichtung gekoppelt ist, die mit zumindest einem Sensor zur Erfassung von Zustandsdaten der Gelenkeinrichtung gekoppelt ist und auf der Grundlage von Sensorwerten des zumindest einen Sensors den Aktuator aktiviert, deaktiviert oder moduliert, und mit zumindest einem Biosignalsensor, der eine Muskelaktivität oder eine Ansteuerung eines Muskels oder mehrerer Muskeln als Biosignal erfasst und der Steuerungseinrichtung übermittelt, wobei auf der Grundlage des Biosignals oder der Biosignale der Aktuator aktiviert, deaktiviert oder moduliert wird, sieht vor, dass die Beeinflussung der Verschwenkbewegung durch den Aktuator gegen eine Flexion, also eine Veränderung des Flexionswiderstandes, auf der Grundlage eines Biosignals erhöht wird und anschließend der erhöhte Widerstand gegen eine Flexion auf der Grundlage von Sensorwerten verringert wird. Die Erhöhung des Flexionswiderstandes bzw. der die verstärkte Beeinflussung der Verschwenkbewegung gegen eine Flexion durch den Aktuator erfolgt auf der Grundlage eines Biosignals, beispielsweise über eine Detektion eine Kontraktion, einer Kokontraktionen oder über die Ableitung von Nervensignalen oder elektromechanisch durch Erfassung einer Längenänderung beispielsweise eines aufgeklebten Sensors oder eines Liners. Somit ist es beispielsweise möglich, ein Gelenk durch eine Kontraktion oder Kokontraktion zu verriegeln. Bei einem Wegfall des Biosignals, beispielsweise durch eine Verringerung der Muskelspannung, findet jedoch keine oder zumindest nicht zwangsweise eine Verringerung des Flexionswiderstandes statt, vielmehr wird der erhöhte Widerstand gegen die Flexion auf der Grundlage von Sensorwerten verringert, also von Sensorwerten, die zur Erfassung von Zustandsdaten der Gelenkeinrichtung ausgebildet und vorgesehen sind. Der erhöhte Flexionswiderstand wird unabhängig von einer Veränderung des Biosignals so lange beibehalten, bis zumindest ein Sensorsignal aufgrund einer Bewegung oder Zustandsänderung in einer definierten Größe von dem zumindest einen Sensor detektiert wird. Somit wird für die Auslösung einer Verringerung des Flexionswiderstandes ein Schwellwert festgelegt, an dessen Erreichen oder Überschreiten oder Unterschreiten die Verringerung geknüpft ist. Beispielsweise muss das Oberteil und/oder das Unterteil mit einer bestimmten Geschwindigkeit bewegt, beispielsweise verschwenkt werden, bevor der Flexionswiderstand wieder verringert wird. Auch können bestimmte Belastungsgrenzen überschritten oder unterschritten werden müssen, damit die Verringerung des Flexionswiderstandes ausgelöst wird. Es ist auch möglich, dass mehrere Grenzwerte unter- oder überschritten werden. Zudem können aus mehreren Eingangssignalen Hilfsgrößen berechnet werden, welche wiederum mit einem oder mehreren Schwellwerten oder Grenzwerten verglichen werden und eine Verringerung des Flexionswiderstandes auslösen. Neben einer Steuerung über Grenzwerte kann der Flexionswiderstand kontinuierlich und/oder in mehreren diskreten Stufen auf Basis der Sensorwerte angepasst werden. Alternativ oder ergänzend können für die Steuerung mittels Biosignalen und/oder Sensoren Algorithmen der Signalverarbeitung, der Statistik, der Klassifikation, des maschinellen Lernens und/oder der künstlichen Intelligenz angewendet werden. Auch können modellbasierte Verfahren für die Steuerung verwendet werden.

In einer Ausgestaltung wird der erhöhte Flexionswiderstand nur auf der Grundlage von Sensorwerten oder auf der Grundlage eine Kombination aus Sensorwerten und einem Biosignal wieder verringert. Somit kann auch bei einer fortgesetzten Aktivierung der Muskulatur eine Verringerung des Flexionswiderstandes und gegebenenfalls einer Unterstützung einer Flexion nur bei Vorliegen entsprechender Zustandsdaten wie Beschleunigung, Winkelstellungen, Orientierungen im Raum, relative oder absolute Positionen, Lageänderung oder Belastungen, ggf. in Verbindung mit einem entsprechenden Biosignal erfolgen. Belastungen können Kräfte, Momente, Drücke, Kraftangriffspunkte und/oder Hebelarme sein. Auch die zeitlichen Ableitungen und Verläufe von Zustandsdaten können für die Steuerung herangezogen werden.

Die Sensoren erfassen in einer Ausgestaltung insbesondere eine Bewegung des Oberteils oder des Unterteils relativ zueinander oder auch absolut.

In einer Ausgestaltung löst das zumindest eine Biosignal unabhängig von dem Zustand der orthopädietechnischen Gelenkeinrichtung, insbesondere unabhängig von der Positionen, Belastung und/oder einer Bewegung der Gelenkeinrichtung oder des Oberteils oder des Unterteils die Erhöhung des Flexionswiderstandes aus. Somit kann in jedem Zustand der orthopädietechnischen Gelenkeinrichtung eine Flexionssperre oder eine Erhöhung des Flexionswiderstandes ausgeführt werden. Dies ist insbesondere vorteilhaft, um bei Notsituationen eine schnelle Verriegelung oder eine schnelle Erhöhung des Flexionswiderstandes zu bewirken. Ein natürlicher Reflex bei unvorhergesehenen Situationen, beispielsweise beim Stolpern, ist eine Anspannung von Muskeln, insbesondere auch eine Kokontraktion. Durch eine solche unwillkürliche Kokontraktion wird ein starkes Biosignal ausgesendet, das als Notverriegelung oder Notfallantwort gewertet werden kann und immer eine Erhöhung des Flexionswiderstandes auslöst.

Alternativ löst das Biosignal die Erhöhung des Flexionswiderstandes nur dann aus, wenn keine oder nur eine unterhalb eines Grenzwertes liegende Bewegung oder Zustandsänderung des Oberteils und/oder des Unterteils durch den zumindest einen Sensor detektiert wird. Alternativ oder ergänzend löst das Biosignal die Erhöhung des Flexionswiderstandes nur innerhalb eines gewissen Positionsbereichs, Absolut- und/oder Relativwinkelbereichs aus, zum Beispiel einem gewissen Ausmaß an Neigung des Unterschenkels relativ zu dem Oberschenkel oder einer externen Bezugsorientierung, z.B. der Schwerkraft oder in einem gewissen Kniewinkelbereich. Eine weitere oder alternative Bedingung ist beispielsweise auch eine Belastung, insbesondere eine Axialbelastung entgegen der Schwerkraftrichtung des Oberteils und/oder des Unterteils, die detektiert werden muss, bevor die Flexionswiderstandserhöhung ausgeführt wird. Damit wird beispielsweise eine Erhöhung des Flexionswiderstandes oder eine Sperrung der orthopädietechnischen Gelenkeinrichtung nur in bestimmten Situationen ermöglicht, beispielsweise wenn eine Prothese, Orthese oder ein Exoskelett belastet wird.

Sensorwerte sind in einer Ausgestaltung mit einem Schwellwert oder mehreren Schwellwerten versehen, der jeweils überschritten oder unterschritten werden muss, bevor die Steuerungseinrichtung eine Verringerung des Flexionswiderstandes nach einer erfolgten Erhöhung ausführt.

Die orthopädietechnische Gelenkeinrichtung mit einem Oberteil und einem Unterteil, die gelenkig aneinander, um eine Schwenkachse verschwenkbar gelagert sind, mit einem Aktuator, der mit dem Oberteil und dem Unterteil gekoppelt ist und eine Verschwenkbewegung des Oberteils relativ zu dem Unterteil beeinflusst, wobei der Aktuator mit einer Steuerungseinrichtung gekoppelt ist, die mit zumindest einem Sensor zur Erfassung von Zustandsdaten der Gelenkeinrichtung gekoppelt ist und auf der Grundlage von Sensorwerten des zumindest einen Sensors den Aktuator aktiviert, deaktiviert oder moduliert und mit zumindest einem Biosignalsensor, der Biosignale erfasst und der Steuerungseinrichtung übermittelt, wobei auf der Grundlage der Biosignale der Aktuator aktiviert, deaktiviert oder moduliert wird, sieht vor, dass die Steuerungseinrichtung eingerichtet ist, die Beeinflussung durch den Aktuator gegen eine Flexion auf der Grundlage eines Biosignals zu erhöhen und den erhöhten Flexionswiderstand auf der Grundlage von Sensorwerten zu verringern, wobei der erhöhte Flexionswiderstand unabhängig von einer Veränderung des Biosignals solange beibehalten wird, bis zumindest ein Sensorsignal aufgrund einer Bewegung, Belastung und/oder Zustandsänderung in einer definierten Größe von dem zumindest einem Sensor (50) detektiert wird. Der Aktuator ist in einer Ausgestaltung als aktiver Aktuator mit einem Antrieb, beispielsweise in Gestalt eines Elektromotors oder eines Kraftspeichers, oder als ein passiver Aktuator, der als Bremse oder Dämpfer wirkt, ausgestaltet.

Die Gelenkeinrichtung kann an einer unteren oder oberen Extremität angeordnet oder ausgebildet sein, insbesondere als ein künstliches Knöchelgelenk, als ein künstliches Kniegelenk, als ein künstliches Hüftgelenk, als ein künstliches Ellenbogengelenk oder als ein künstliches Schultergelenk.

Der zumindest eine Sensor zur Erfassung von Zustandsdaten oder Bewegungsdaten der orthopädietechnischen Gelenkeinrichtung ist an dem Oberteil und/oder dem Unterteil angeordnet und erfasst insbesondere Stellungen im Raum, Stellungen von Oberteil und Unterteil zueinander, Kräfte, Momente, Beschleunigungen, Geschwindigkeiten und Veränderungen von Zuständen.

Biosignale sind jegliche biologisch generierten Signale, die die willentliche oder unwillkürliche Ansteuerung der orthopädietechnischen Gelenkeinrichtung ermöglichen. Aus Biosignalen kann auf die Ansteuerung von einem oder mehreren Muskeln geschlossen werden. Bei der Ansteuerung von einem oder mehreren Muskeln kann es sich auch um die Intention handeln diese anzusteuern, insbesondere dann, wenn die anzusteuernde Muskulatur nicht mehr vorhanden oder nicht mehr ansteuerbar ist, zum Beispiel durch eine Lähmung. Bei einem Biosignal kann es sich um ein elektrisches Signal handeln, zum Beispiel Nervensignale von peripheren Nerven, elektrische Signale bei Kontraktion der Muskulatur (EMG, Elektromyographie) aber auch Signale des zentralen Nervensystems. Elektrische Signale können über Ableiterelektroden erfasst werden. Sind Informationen in einem Signal kodiert, kann zunächst eine Dekodierung erfolgen, bevor das Signal für die Steuerung herangezogen wird. Ein Biosignal kann ein chemisches oder elektrochemisches Signal sein, zum Beispiel die Konzentration eines Stoffes, die Wechselwirkung von Molekülen oder ein elektrochemischer Gradient. Diese Größen können zum Beispiel über die Wechselwirkung mit Licht, wie Absorption oder die Anregung mittels Licht und anschließende Emission, bestimmt werden. Biosignale können auch Leitfähigkeiten von Geweben und/oder Körperteilen sein. Bei Biosignalen kann es sich auch um mechanische Parameter wie Kraft, Druck, Länge und/oder Länge sowie deren zeitliche Veränderungen handeln, beispielsweise der Puls oder die Längenänderung eines Muskels oder von Strukturen innerhalb des Muskels. Diese Größen können zum Beispiel durch Drucksensoren in einem Schaft oder Manschetten bestimmt werden, welche die Verdickung eines Muskels bei dessen Kontraktion erfasst. Längen- und Geometrieänderungen können auch über Ultraschall bestimmt werden. Biosignale können invasiv oder nichtinvasiv erfasst werden. Invasive Sensoren sind beispielsweise implantierte myoelektrische Elektroden, um einen Nerv gelegte Elektroden oder Nadelelektroden im Gehirn oder Rückenmark. Signale invasiver Sensoren können drahtlos nach außen kommunizieren, um einen Hautdurchtritt zu vermeiden. Auch die Energieversorgung kann drahtlos mittels induktiver Energieversorgung ermöglicht werden. Bei osseointegrativen Versorgungen mittels eines Ende-Exo-Implantats kann die Anbindung der Sensoren über das Implantat erfolgen. Es ist auch möglich, dass Biosensoren teilweise invasiv sind. Zum Beispiel können Magnetkörper invasiv in das Gewebe, zum Beispiel den Muskel oder eine Sehne, eingebracht und die Verschiebung der Magnete zueinander bei Bewegung oder Anspannung der Muskulatur durch externe Sensoren erfasst werden. Es können auch mehrere Verfahren kombiniert werden oder verschiedene Biosignale erfasst werden, insbesondere um eine höhere Robustheit gegenüber Störungen und Fehlerkennungen zu erzielen. **In** einer Ausgestaltung basiert die Steuerung der orthopädietechnischen Einrichtung auf zumindest einem Biosignals das mit einer physischen oder intendierten Muskelanspannung, Kontraktion und/oder Ansteuerung des Bewegungsapparates in Verbindung steht, insbesondere über Elektromyographie. Durch ein solches Biosignal ist es der tragenden Person möglich Einfluss auf die Steuerung zu nehmen. Es können unterschiedliche Filter und Algorithmen der Signalverarbeitung auf die erfassen Biosignale angewendet und die resultierenden Größen für die Steuerung herangezogen werden. Damit ist es zum Beispiel möglich eine Ansteuerung über ein Biosignal nur dann vorzunehmen, wenn das Biosignal bestimmte Kriterien erfüllt, bspw. ein gewisses Spektrum oder einen gewissen zeitlichen Verlauf aufweist. Zum Beispiel kann ein hochfrequentes Wechselsignal tiefpassgefiltert und gleichgerichtet und dieses Signal dann für die Steuerung herangezogen werden. Neben dem Biosignal können auch errechnete Größen wie zeitliche Ableitungen, integrale Größen, statistische Größen oder Eigenschaften im Frequenzbereich für die Steuerung herangezogen werden.

In einer Ausgestaltung wird die Ansteuerung von einem Muskel oder mehreren Muskeln ermittelt und für die Steuerung herangezogen. Zum Beispiel kann bei einer Knieprothese oder -orthese eine Ableiterelektrode auf der Vorderseite des Oberschenkels platziert werden, welche die Kontraktion des Musculus Quadrizeps Femuris oder des Musculus Biceps Femuris erfasst und dieses Biosignal für die Steuerung herangezogen werden. Eine Steuerung über nur einen Muskel ist besonders einfach und kostengünstig. Es ist auch möglich, dass die Kontraktion mehrerer Muskeln erfasst wird, aber nur die Kontraktion von einem Muskel oder einem Teil der Muskeln für die Steuerung herangezogen werden. Bei einer Steuerung über mehrere Muskeln kann eine Kokontraktion, das gleichzeitige Anspannen von mehreren Muskeln, von zwei oder mehr Muskeln ermittelt und für die Steuerung herangezogen werden. Bei einer Kokontraktion muss es sich nicht zwangsweise um die Kontraktion von Agonist und Antagonist handeln. Es ist jedoch auch möglich, in unterschiedlichen Situationen, Bewegungsphasen oder Modi unterschiedliche Muskelaktivierungen oder unterschiedliche Muskeln und/oder Muskelgruppen für die Steuerung heranzuziehen. In einigen Bewegungssituationen werden bestimmte Muskeln unwillkürlich angespannt, wodurch aufgrund der Muskelaktivierung nicht mehr eindeutig darauf geschlossen werden kann, ob eine Erhöhung eines Widerstandes erfolgen soll. Auch sind einige Muskeln gelenksübergreifend, zum Beispiel gleichzeitig hüftstreckend und kniebeugend, wodurch bei einer Hüftstreckung zwangsweise auch ein Kniestreckmoment aufgebracht wird. Diese Synergie kann für eine besonders intuitive Steuerung genutzt werden.

In einer Ausgestaltung werden Verfahren des maschinellen Lernens und/oder der künstlichen Intelligenz auf die Biosignale angewendet und die daraus gewonnenen kontinuierlichen oder diskreten Informationen, wie Klassen, für die Steuerung herangezogen. Mittels Verfahren der Mustererkennung können unterschiedliche Signal-Merkmale von einem Biosignal oder mehreren Biosignalen verwendet werden, um auf eine spezifische Ansteuerung zu schließen, zum Beispiel auf ein spezifisches Aktivierungsmuster mehrerer Muskeln oder die Unterscheidung zwischen einer willentlichen oder unwillkürlichen Muskelanspannung. Damit ist es möglich eine Widerstandserhöhung und/oder -reduktion nur dann durchzuführen, wenn ein spezifisches Signal-Muster vorliegt. Algorithmen des maschinellen Lernens und/oder der künstlichen Intelligenz können alternativ oder ergänzend auch für die Veränderung der Widerstände und damit auch auf die anderen Sensorwerte angewendet werden.

**In** einer Ausgestaltung ist die Steuerung mittels des zumindest einen Biosignals abhängig von einer Situation, einem Bewegungsmuster, einer Bewegungsphase, und/oder einem Modus der orthopädietechnischen Einrichtung. Zum Beispiel kann die Steuerung mittels Biosignal in einem Sondermodus für das Fahrradfahren anders gestaltet sein als in einem Modus für das Gehen. Es ist auch möglich, dass die Steuerung vom aktuellen Bewegungsmusters abhängt, zum Beispiel dass beim Geradeausgehen eine anderer Steuerung aktiv ist als beim Gehen um eine Kurve oder dass beim schnellen Gehen eine andere Steuerung aktiv ist als beim langsamen Gehen. Auch in Abhängigkeit der Bewegungsphase kann die Steuerung verändert werden. Zum Beispiel kann eine andere Steuerung in der terminalen Standphase als in der frühen Schwungphase erfolgen. Die Bewegungsphasen können über die Sensoren ermittelt werden. Es ist auch möglich, dass in speziellen Modi, Bewegungsmustern oder Bewegungsphasen keine Steuerung mittels Biosignal aktiv ist. Nicht zuletzt ist es möglich, dass die anwendende Person über Interfaces, wie Bedienelemente oder eine App, die Steuerung mittels Biosignalen temporär oder dauerhaft aktivieren und/oder deaktivieren kann. Die Anpassung in Abhängigkeit der Situation, einem Bewegungsmuster, einer Bewegungsphase und/oder einem Modus kann sowohl die Kriterien für die Widerstandserhöhung und/oder -verringerung auf Basis des Biosignals betreffen als auch Parameter der Widerstandserhöhung und/oder -verringerung, wie zum Beispiel eine Sensitivität oder Verstärkung der Widerstandspassung in Abhängigkeit des Biosignals, das Ausmaß der Widerstandsanpassung oder die Art der Widerstandsanpassung, wie eine Änderung einer Dämpfung, einer Steifigkeit, einer Gleichgewichtslage und/oder eines Moments.

**In** einer Ausgestaltung wird neben einem Flexionswiderstand auch zumindest ein Extensionswiderstand auf Grundlage des zumindest einen Biosignals erhöht, gegebenenfalls bis hin zu einer Sperre. Die Kriterien für die Erhöhung und Absenkung des Extensionswiderstands sowie die beeinflussten Parameter der Steuerung können von jenen des Flexionswiderstandes abweichen.

**In** einer Ausgestaltung bleibt ein Flexionswiderstand bei Ansteuerung über ein Biosignal erhöht. Die Erhöhung des Flexionswiderstandes bezieht sich dabei auf den Widerstand, der ohne Ansteuerung über ein Biosignal vorliegen würde. Bei einer konventionellen Stehfunktion eines Prothesenkniegelenks, bei der die Beugung im Stehen zum Beispiel vollständig gesperrt ist, wird beispielsweise ein Flexionswiderstand reduziert, wenn die Prothese entlastet, das Kniegelenk gestreckt oder das Bein schnell nach vorne oder hinten rotiert wird. Obwohl dieses Verhalten in vielen Situationen vorteilhaft ist, kann es in anderen nachteilig sein. Entsprechend kann in einer Ausgestaltung bei einer Ansteuerung über das Biosignal der Widerstand erhöht oder gegebenenfalls gesperrt bleiben, obwohl auf Grundlage von anderen Sensoren eine Reduktion eines Flexionswiderstandes erfolgen würde. Das Biosignal überstimmt oder overruled in diesem Fall die anderen Sensoren.

In einer Ausgestaltung ist der Verlauf der Erhöhung und/oder Verringerung des Flexionswiderstandes zeitgesteuert und/oder in der Änderungsrate limitiert. Insbesondere bei der Reduktion des Flexionswiderstandes auf Basis des zumindest einen Biosignals und/oder anderen Sensoren kann ein abruptes Reduzieren des Widerstandes als unangenehmen empfunden werden oder sogar unsicher sein. Entsprechend ist es vorteilhaft den Widerstand nicht schlagartig zu erhöhen oder zu reduzieren, sondern kontinuierlich mit einer reduzierten oder beschränkten Änderungsrate, auch dann, wenn die zugrundeliegenden Biosignale sich mit sehr hoher Änderungsrate beziehungsweise abrupt ändern. Zum Beispiel kann ein zeitlicher Verlauf für die Änderungen zwischen dem erhöhten und dem normalen Flexionswiderstand in der Steuerung hinterlegt sein und angewendet werden oder eine Tiefpassfilterung durchlaufen.

In einer Ausgestaltung hängt die Änderung des Flexionswiderstandes, insbesondere das Ausmaß der Erhöhung und/oder Reduktion des Widerstandes, auch von anderen Größen ab, die zum Beispiel über Sensoren erfasst werden, insbesondere von Relativ- und Segmentwinkeln, Belastungen und/oder deren zeitlichen Ableitungen und/oder Verläufen. Zum Beispiel kann die Erhöhung des Flexionswiderstandes von einem Kniewinkel, einem Unterschenkelwinkel, einer Verschwenkgeschwindigkeit oder auch einem Knöchel- oder Kniemoment abhängen.

Die beschriebenen Steuerungsverfahren können auch auf einen Fuß mit beweglichem Knöchelgelenk oder eine Hüftgelenk angewendet werden. Es können auch mehrere Achsen eines Gelenks gesteuert werden, zum Beispiel sowohl die Flexion und Extension als auch die Adduktion und Abduktion eines Hüftgelenks. Bei einem Fuß ist vor allem ein Anheben eines Widerstands gegen eine Dorsalflexion für das Stehen sinnvoll. Auf Basis von einem oder mehreren Biosignalen kann ein Widerstand gegen eine Dorsalflexion angehoben werden, wobei bei einem Wegfall des Biosignals der Dorsalflexionswiderstand nicht zwangsweise reduziert wird, sondern dies auf Basis von Sensorwerten erfolgt, also von Sensorwerten, die zur Erfassung von Zustandsdaten der Gelenkeinrichtung ausgebildet sind. Bei einem Hüftgelenk ist vor allem die Anhebung des Widerstands gegen eine Hüftflexion für das Stehen vorteilhaft. Es können auch die Widerstände mehrerer Gelenke gleichzeitig angesteuert werden.

**In** einer Ausgestaltung wird der Flexionswiderstand über einen oder mehrere Impulse des zumindest einen Biosignals erhöht. Dabei erfolgt nur eine kurze Ansteuerung über das Biosignal, wobei der Flexionswiderstand nach Wegfall des Biosignals zunächst erhalten bleibt und die Reduktion des Flexionswiderstands im Weiteren auf Basis von Sensorwerten erfolgt.

**In** einer Ausgestaltung wird der Flexionswiderstand in einem nichtbelasteten Zustand der orthopädietechnischen Einrichtung auf Basis zumindest eines Biosignals erhöht. Dies kann nützlich sein, um zum Beispiel das mit einem künstlichen Kniegelenk versorgte Bein zu repositionieren, zum Beispiel um in ein Auto einzusteigen. Ohne den erhöhten Flexionswiderstand würde das Kniegelenk bei einem nach vorne Anheben des Beines durch die auf den Unterschenkel und Fuß wirkende Schwerkraft gebeugt werden.

**In** einer Ausgestaltung wird der durch das Biosignal erhöhte Flexionswiderstand wieder reduziert, wenn ein gewisser Bereich einer Winkellage, eine Belastung und/oder einer Geschwindigkeit überschritten wird oder nach einer definierten Zeit. Bei einer Beinprothese oder Orthese ist es aus Sicherheitsgründen zum Beispiel sinnvoll einen Flexionswiderstand in einem Kniegelenk zu reduzieren, wenn eine besonders starke Rückwärts- oder Vorwärtsneigung des Beins erkannt wird oder das Bein besonders schnell nach vorne oder hinten rotiert. Auch bei einer besonders hohen Belastung kann es aus Sicherheitsgründen sinnvoll sein den Flexionswiderstand zu reduzieren, um eine Überlastung der Anbindung an den Körper oder des Körpers selber zu verhindern. **In** solchen Situationen kann es zu einer unwillkürlichen Ansteuerung über das Biosignal durch die nutzende Person kommen, welche die Reduktion über Sensorsignale notwendig macht.

**In** einer Ausgestaltung wird der erhöhte Flexionswidertand bei aufrechterhaltener Ansteuerung über das Biosignals auf Basis von Sensoren nur teilweise reduziert. Erst wenn auch die Ansteuerung über das Biosignal verringert wird, kommt es zur vollständigen Reduktion des Flexionswiderstandes auf ein entsprechendes Widerstandsniveau. Durch eine solche Ansteuerung kann ein Flexionswiderstand durch die Ansteuerung mittels Biosignal teilweise erhöht bleiben, obwohl auf Basis der anderen Sensoren eine Verringerung des Widerstandes in einem größeren Ausmaß erfolgen würde.

Die Erhöhung des Flexionswiderstandes kann von einem hohen, nicht sperrenden Widerstand ausgehen, wie er zum Beispiel für eine Beinprothese zum stabilen, aufrechten Stehen oder während der Standphase des Gehens auf unterschiedlichen Untergründen und Untergrundneigungen vorteilhaft ist. Es ist auch möglich, dass eine Erhöhung ausgehend von einem sehr niedrigen oder minimalen Widerstand erfolgt, wie er zum Beispiel in der Schwungphase einer Beinprothese oder bei einer entlasteten Beinprothese vorliegt. Wird der Flexionswiderstand durch die Ansteuerung über ein Biosignal erhöht, kann auf Basis von Sensordaten wieder eine Reduktion auf das Ausgangsniveau erfolgen. Alternativ oder ergänzend kann eine Reduktion auf einen minimalen Flexionswiderstand erfolgen. Dies ist insbesondere bei einer Beinprothese oder -orthese in der terminalen Standphase oder bei Entlastung von Vorteil, um eine leichte Einleitung der Schwungphase zu ermöglichen oder eine Beugebewegung aktiv zu unterstützen. Die Reduktion kann trotz anhaltender Aktivierung durch das Biosignal auf Basis von Zustandsdaten der Prothese oder Orthese, die über Sensoren ermittelt werden, erfolgen, zum Beispiel auf Basis einer Vorwärtsneigung eines Unterschenkels und einer Belastung auf dem Vorfuß.

Nachfolgend werden Ausführungsbeispiele der Erfindung anhand der Figuren näher erläutert. Es zeigen:
Figur 1 eine schematische Darstellung einer orthopädietechnischen Gelenkeinrichtung;
Figuren 2 bis 6 -verschiedene Nutzungssituationen;
Figur 7 - eine schematische Darstellung der Steuerungsfunktion;
Figuren 8 bis 12 - verschiedene Steuerungsschemata;
Figur 13 - Korrelationen zwischen Biosignal und Widerstandsparameter;
Figur 14 - schematische Darstellungen einer Widerstandserhöhung; sowie
Figur 15 - eine weitere Nutzungssituation.

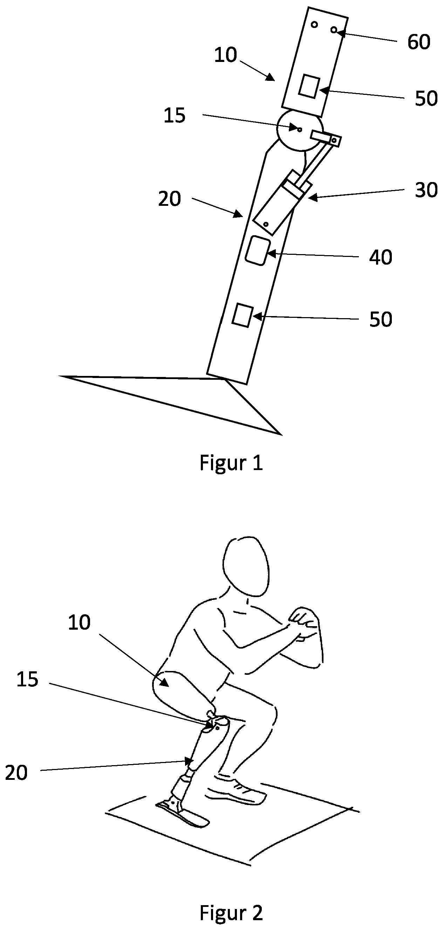

**In** der Figur 1 ist in einer schematischen Darstellung eine orthopädietechnische Gelenkeinrichtung in Gestalt einer Prothese der unteren Extremität dargestellt. Die orthopädietechnische Gelenkeinrichtung weist ein Oberteil 10 und ein Unterteil 20 auf, die schwenkbar um eine Schwenkachse 15 aneinander gelagert sind. Zur Beeinflussung der relativen Verschwenkung des Oberteils 10 zu dem Unterteil 20 um die Schwenkachse 15 ist an dem Oberteil 10 und an dem Unterteil 20 ein Aktuator 30 angeordnet, der in dem dargestellten Ausführungsbeispiel als ein Hydraulikdämpfer ausgestaltet ist. **In** alternativen Ausgestaltungen kann der Aktuator 30 als ein aktiver Aktuator mit einem Antrieb, beispielsweise einem Elektromotor, einem Kraftspeicher oder einer anderen Art und Weise des Antriebes ausgestattet sein, um eine Verschwenkbewegung des Oberteils 10 relativ zu dem Unterteil 20 zu beeinflussen. **In** dem dargestellten Ausführungsbeispiel ist der Aktuator 30 als passiver Aktuator ausgebildet und stellt einen Widerstand gegen eine Flexionsbewegung und gegebenenfalls einer Extensionsbewegung zur Verfügung. Der Aktuator 30 ist mit einer Steuerungseinrichtung 40 gekoppelt, die in dem dargestellten Ausführungsbeispiel an dem Unterteil 20 angeordnet ist. Die Steuerungseinrichtung 40 ist mit Sensoren 50 gekoppelt, die an dem Oberteil 10 und/oder dem Unterteil 20 angeordnet sind. Die Sensoren 50 erfassen Zustandsgrößen der Gelenkeinrichtung, z.B. Positionen, Lagen, Kräfte, Momente, Beschleunigungen oder Stellungen von Komponenten im Raum oder zueinander, wobei eine Vielzahl von Sensoren eingesetzt werden kann, um die gewünschten Zustandsgrößen der orthopädietechnischen Gelenkeinrichtung zu erfassen. Die Sensoren 50 sind mit der Steuerungseinrichtung 40 gekoppelt und übermitteln entsprechende Sensordaten oder Sensorsignale an die Steuerungseinrichtung 40, wobei auf Grundlage der Sensorwerte oder Sensordaten der Aktuator 30 hinsichtlich seiner Fähigkeit, die Verschwenkbewegung oder Verschwenkbarkeit des Oberteil 10 relativ zu dem Unterteil 20 zu beeinflussen, verändert wird. Beispielsweise wird der Flexionswiderstand und/oder Extensionswiderstand in Abhängigkeit von den Sensorwerten verändert. Dazu werden Ventile oder Drosseln verstellt. In alternativen Ausführungsform können Magnetfelder verändert werden, um magnetorheologische Fluide hinsichtlich ihrer Viskosität zu verändern. Bei einer mechanischen Bremseinrichtung können Bremskräfte erhöht oder verringert werden, um ein angepasstes Widerstandsverhalten des Aktuators 30 zu erzeugen. Bei einem aktiven Antrieb, der zumindest einen elektromechanischen Aktuator aufweist, können über Ströme und Spannungen Widerstände gegen eine Bewegung aufgebracht oder aber auch Bewegungen unterstützt werden. Neben dem Aufbringen von Momenten und Momentenverläufen können über Steuerungsalgorithmen und Sensorinformationen über die Verschwenkbewegung von Oberteil und Unterteil Trajektorien verfolgt oder Systemeigenschaften im Sinne einer Impedanz- oder Admittanz-Regelung emuliert werden. Zum Beispiel kann das Verhalten einer linearen oder nichtlinearen Feder, das Verhalten eines Dämpfers oder eine Trägheit emuliert werden oder eine Kombination mehrerer Eigenschaften, wodurch eine Verschwenkbewegung entgegen einer Flexion beeinflusst werden kann. Eine solche Ansteuerung bietet ein hohes Maß an Flexibilität. Durch derartige Steuerungen können Verschwenkbewegungen auch aktiv unterstützt werden. Wird über einen Aktuator ein Moment oder eine Kraft aufgebracht, ohne dass eine Verschwenkung stattfindet, bspw. weil externe und interne Kräfte im Gleichgewicht stehen, wird der Verschwenkbewegung ebenfalls ein Widerstand entgegengebracht. Über Aktuatoren können auch Energiespeicher aktiviert und deaktiviert werden, zum Beispiel ein hydraulischer Federspeicher, Übersetzungen von Antrieben verändert und/oder diese ein- oder ausgekoppelt werden. Diese Arten der Aktuierung können ebenfalls zur Beeinflussung der Verschwenkbewegung angewendet werden. Widerstände sind Kräfte und Momente, die durch Aktuatoren aufgebracht werden, um eine Verschwenkbewegung zu beeinflussen. Ein Widerstand kann einer Verschwenkbewegung entgegengesetzt sein, aber auch eine Unterstützung einer Verschwenkbewegung sein, das heißt, in Richtung der Verschwenkung wirken.

An dem Oberteil 10 ausgebildet oder daran befestigt, beispielsweise an einem Prothesenschaft oder an einer Halterung, sind Biosignalsensoren 60, die zur Erfassung von Muskelaktivitäten oder einer Ansteuerung eines Muskels oder der Muskulatur ausgebildet sind und Biosignale erzeugen, die an die Steuerungseinrichtung 40 übermittelt werden. Die Biosignalsensoren 60, die als Ableiterelektroden ausgebildet sein können, sind mit der Steuerungseinrichtung 40 über eine leitende Verbindung oder drahtlos gekoppelt und übermitteln entsprechende Signale an die Steuerungseinrichtung 40, die den Aktuator 30 ebenfalls auf der Grundlage der Biosignale aktiviert, deaktiviert oder moduliert, um den Widerstand zu verändern oder das Bewegungsverhalten oder die Verlagerbarkeit der Komponenten zueinander zu beeinflussen.

Abweichend von der Ausgestaltung der orthopädietechnischen Gelenkeinrichtung als Prothese kann diese auch als Orthese ausgebildet sein, wobei statt des Unterschenkelteils als Unterteil 20 eine Unterschenkelschiene an einem künstlichen Kniegelenk ausgebildet ist. Das Oberteil 10 ist dann eine Oberschenkelschiene, die über entsprechende Befestigungseinrichtungen wie Gurten, Schalen oder Manschetten an dem Oberschenkel festgelegt werden kann. Die Biosignalsensoren 60 sind entweder separat an dem Oberschenkel oder einer anderen Muskulaturgruppe befestigt und können auch an der Befestigungseinrichtung angeordnet sein. Die orthopädietechnische Gelenkeinrichtung kann auch als Exoskelett als Sonderform einer Orthese ausgebildet sein. Alternativ zu der dargestellten Ausführungsform als orthopädietechnische Gelenkeinrichtung der unteren Extremität kann die orthopädietechnische Gelenkeinrichtung auch für eine obere Extremität ausgebildet sein, beispielsweise als künstliches Schultergelenk oder künstliches Ellenbogengelenk für einen Prothesenarm oder als Orthese bzw. ein Exoskelett für einen Arm.

Die Aktivierung, Deaktivierung und gegebenenfalls das Modulieren des Aktuators zur Beeinflussung einer Verschwenkbewegung oder der Verschwenkbarkeit der Gelenkeinrichtung erfolgt auf der Grundlage zumindest eines Biosignals von dem Biosignalsensor dergestalt, dass die Beeinflussung der Verschwenkbewegung oder Verschwenkbarkeit gegen eine Flexion erhöht wird, wenn ein entsprechendes Biosignal vorliegt. Unter einer Beeinflussung der Verschwenkbewegung wird auch verstanden, dass eine Verschwenkbewegung gesperrt wird, sodass das Unterteil 20 relativ zu dem Oberteil 10 verriegelt wird und keine Relativbewegung zwischen den beiden Teilen stattfindet und stattfinden kann. Die Beeinflussung der Verschwenkbarkeit des Oberteils 10 relativ zu dem Unterteil 20 kann also in einer Sperrung des Gelenks in Flexionsrichtung oder einer Erhöhung des Flexionswiderstandes erfolgen. Die Beeinflussung wird entweder durch einen passiven oder einen aktiven Aktuator durchgeführt. Um den erhöhten Flexionswiderstand der Gelenkeinrichtung wieder zu verringern, erfolgt dies auf der Grundlage von Sensorwerten, also von Werten, die von dem Sensor 50 oder den Sensoren 50 an die Steuerungseinrichtung 40 übermittelt werden, ggf. in Verbindung mit oder in Abhängigkeit von einem Biosignal. Eine solche Steuerung der orthopädietechnischen Gelenkeinrichtung, beispielsweise einer unteren Extremität ist beispielsweise für eine Stehfunktion vorteilhaft und sinnvoll, bei der eine Flexion, beispielsweise des Kniegelenkes, erschwert oder gesperrt werden soll.

In der Figur 2 ist eine nutzende Person einer orthopädietechnischen Gelenkeinrichtung einer unteren Extremität mit einem Oberteil 10, das einen Prothesenschaft aufweist, und einem Unterteil 20 in Gestalt eines Unterschenkelteils mit einem distal daran befestigten Prothesenfuß gezeigt. Das Oberteil 10 ist relativ zu dem Unterteil 20 um die Schwenkachse 15 verschwenkbar. Der Aktuator, wie er in der Figur 1 dargestellt ist, ist in dem Unterteil 20 angeordnet und an dem Oberteil 10 mit seinem oberen Ende befestigt. Die nutzende Person der orthopädietechnischen Gelenkeinrichtung befindet sich in einer tiefen Hocke, in der beide Beine belastet sind. Aus dem Stand der Technik ist bekannt, dass bei einem gebeugten, ruhigen Stehen, das über eine Erfassung von dem Kniewinkel erkannt wird, der Widerstand gegen eine Flexion erhöht wird, bis ein entspanntes Stehen möglich ist. Dies erfolgt beispielsweise durch eine Sperrung des Kniegelenks oder durch das Aufbringen eines Streckmomentes. Um der Steuerungseinrichtung zu signalisieren, dass eine entsprechende Funktion aktiviert werden soll, muss die anwendende Person ein vordefiniertes Standardverhalten zeigen und die orthopädietechnische Gelenkeinrichtung in einem bestimmten Zustand halten, beispielsweise eine ruhige Gleichgewichtsposition einnehmen. Dazu sind ein erhöhtes Streckmoment und gegebenenfalls eine teilweise Entlastung der mit der orthopädietechnischen Gelenkeinrichtung versorgten Seite notwendig. Das Einnehmen einer solchen Position ist in manchen Stellungen, wie beispielsweise in der dargestellten tiefen Hocke, bei einer hohen Belastung der versorgten Seite oder bei einer Belastung des Vorderfußes schwer bis unmöglich. Es ist somit für eine nutzende Person mitunter schwer, überhaupt die notwendigen Voraussetzungen zu erfüllen, damit die Stehfunktion auf herkömmliche Art und Weise aktiviert wird. Dies ist insbesondere bei instabilen Positionen schwierig, koordinativ anspruchsvoll und anstrengend.

Das vorgeschlagene Verfahren sieht vor, dass eine Aktivierung der Stehfunktionen mit einem erhöhten Widerstand gegen eine Flexion über zumindest ein Biosignal erkannt wird, das durch den Biosignalsensor oder die Biosignalsensoren erfasst und der Steuerungseinrichtung übermittelt wird. Durch das Biosignal oder mehrere Biosignale kann die Flexionsbewegung in Situationen gestoppt oder verlangsamt werden, in denen es einer nutzenden Person nicht möglich ist, über einen gewissen Zeitraum eine notwendige Gleichgewichtsposition einzunehmen. Durch die Aktivierung oder das Modulieren des Aktuators aufgrund zumindest eines Biosignals ist es mitunter erst möglich, die ruhige Stehposition einzunehmen oder das Einnehmen der Stehposition zu erleichtern. Sobald die gewünschte Position erreicht ist, kann der erhöhte Widerstand gegen eine Flexion aktiv bleiben, auch ohne das weitere Vorliegen zumindest eines Biosignals. Eine nutzende Person kann somit in der dargestellten tiefen Hocke wesentlich leichter stehen und verbleiben.

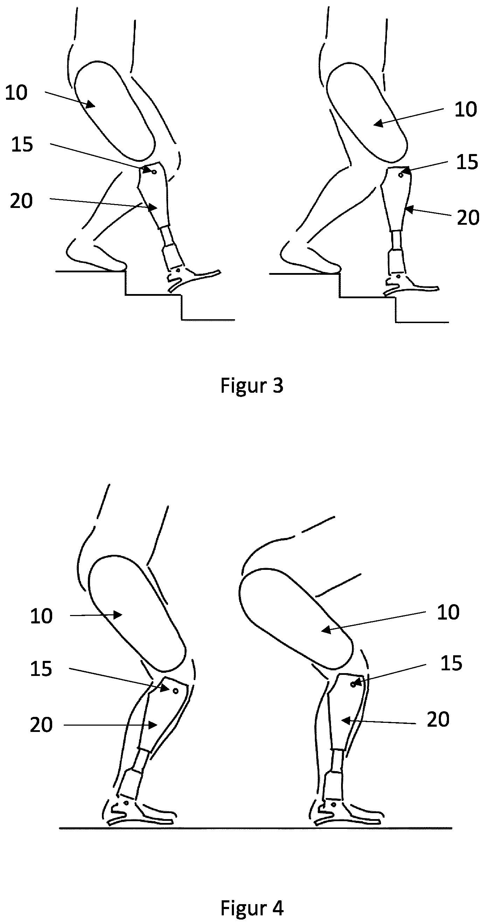

In der Figur 3 ist eine andere Nutzungssituation einer orthopädietechnischen Gelenkeinrichtung dargestellt, nämlich das Herabgehen einer Treppe. Bei dem Absetzen des Prothesenfußes auf einer tieferen Stufe ist es besonders schwer bis unmöglich, diese Bewegung anzuhalten und eine ruhige Stehposition beizubehalten, weil ein hohes Flexionsmoment um die Schwenkachse 15 aufgebracht wird und keine Stabilisierung über den Prothesenfuß erfolgen kann, weil dieser nur mit der Ferse auf der Vorderkante der unteren Treppenstufe aufsetzt. Der Prothesenfuß rollt über die Kante der Treppenstufe ab. Eine Stehfunktion in dieser Situation auf herkömmliche Weise zu aktivieren, ist für einen Nutzer nicht möglich oder nur außerordentlich schwer möglich. Durch Aktivierung eines Biosignals, beispielsweise einer Muskelkontraktion oder einer Muskelkokontraktion, werden entsprechende Sensorwerte von den Biosignalsensoren oder Ableiterelektroden erfasst und an die Steuerungseinrichtung übermittelt und einer Knieflexion bzw. einer weiteren Knieflexion wird ein erhöhter Flexionswiderstand entgegengesetzt, ggf. bis zu einer Sperrung des Gelenkes. Eine solche Steuerung und eine Erhöhung des Widerstandes gegen eine Flexion kann dynamisch beim Gehen auf der Treppe erfolgen, insbesondere beim Verlangsamen oder Abstoppen auf einer Treppe oder Neigung, alternativ oder ergänzend auch beim einfachen Stehen auf der Kante oder an einer abschüssigen Stelle, beispielsweise auf einem Vorsprung, einem Stein, einer erhöhten Stelle oder einem Podest. Die Erhöhung des Widerstandes ist in der rechten Darstellung der Figur 3 abgeschlossen, durch eine erneute Bewegung der versorgten Seite mit der orthopädietechnischen Gelenkeinrichtung ist es möglich, diesen erhöhten Flexionswiderstand oder die Sperrung des Gelenkes aufzuheben und die orthopädietechnische Gelenkeinrichtung in einem anderen Modus zu betreiben. Wird die Ansteuerung über das Biosignal zu einem Zeitpunkt beendet, zu dem sich die versorgte Seite noch nicht hinreichend in Ruhe befindet und die Kriterien für die Aktivierung einer konventionellen Stehfunktion entsprechend nicht erfüllt sind, wird der Widerstand mit der abnehmenden Ansteuerung über das Biosignal wieder reduziert.

In der Figur 4 ist eine weitere Anwendungsmöglichkeit dargestellt, bei der ein Nutzer der orthopädietechnischen Gelenkeinrichtung in einer eingebeugten Stellung steht, wie es in der linken Zeichnung der Figur 4 dargestellt ist. In manchen Situationen wird die Körperposition verändert, ohne dass ein Kniewinkel geändert wird, beispielsweise indem der Nutzer sich weiter nach vorne beugt. Bei herkömmlichen Steuerungsverfahren wird die bereits eingenommene Stehfunktion zunächst deaktiviert, wodurch der Flexionswiderstand verringert wird. Dies erfolgt auf der Grundlage bestimmter Sensorwerte und weiterer Parameter, beispielsweise nur bei einer Kniestreckung, einer schnellen Bewegung des Oberteils oder Unterteils oder einer vollständiger Entlastung. Über eine Ansteuerung des Aktuators über Biosignale kann die Stehfunktion mit einem erhöhten Widerstand aktiv bleiben, wenn während der Bewegung des Oberkörpers oder des Oberteils oder Unterteils eine Deaktivierung bzw. Ansteuerung aufgrund der Sensorsignale erfolgen würde. Der Nutzer steht, wie in der linken Figur dargestellt, zunächst entspannt in gebeugter Stellung. Während der Positionsänderung des Oberkörpers wird die orthopädietechnische Gelenkeinrichtung über ein Biosignal angesteuert, beispielsweise durch eine Kontraktion der Oberschenkelmuskulatur, die über Biosignalsensoren oder Ableiterelektroden in Gestalt von elektrischen Signalen erfasst und als Biosignale an die Steuerungseinrichtung weitergeleitet werden. Die orthopädietechnische Gelenkeinrichtung verbleibt in der Stehfunktion. Nach der Entspannung der Oberschenkelmuskulatur liegt kein Biosignal mehr an der Steuerungseinrichtung an, der Widerstand über den Aktuator bleibt jedoch weiterhin hoch, da sich der Nutzer weiterhin in einer stehenden, ruhigen Position befindet. Erst wenn eine Bewegung der versorgten Seite erfolgt, wird die Stehfunktion aufgehoben und der Flexionswiderstand durch den Aktuator verringert.

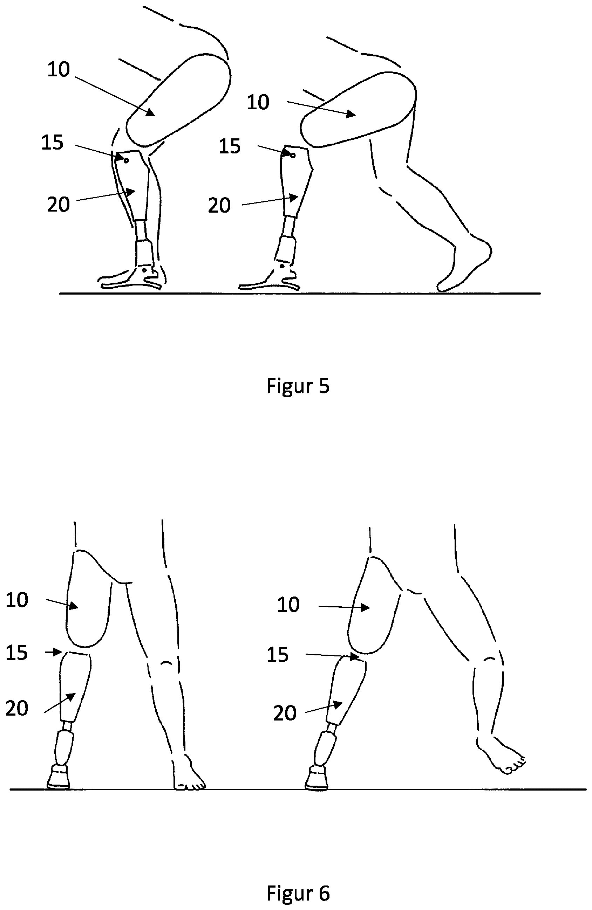

Eine weitere Situation ist in der Figur 5 gezeigt, bei der ein Nutzer der orthopädietechnischen Gelenkeinrichtung einen Gegenstand, der nicht dargestellt ist, zieht. Ausgehend von einer eingebeugten Stellung, die in der linken Zeichnung dargestellt ist, tritt die Person mit der unversorgten, gesunden Seite zurück, um einen vor ihm liegenden Gegenstand zu ziehen oder Zug auszuüben. Eine herkömmliche Stehfunktion würde hier deaktiviert werden, weil die versorgten Seite nach hinten rotiert, also das Unterteil 20 eine Verschwenkbewegung um die Knöchelgelenksachse in rückwärtige, posteriore Richtung ausführt. Wird jedoch ein Biosignal über die Biosignalsensoren der Steuerungseinrichtung übermittelt, ist es möglich, die Stehfunktion willentlich auch während der Rückwärtsbewegung oder rückwärtigen Verschwenkung des Unterteils 20 um eine Knöchelgelenksachse aktiv zu halten. Die Ansteuerung über das Biosignal kann auch in der Position der rechten Darstellung aufrecht gehalten werden, zum Beispiel, wenn die Person sichergehen möchte, dass die Stehfunktion trotz einer Bewegung der Komponenten der Gelenkeinrichtung weiterhin aktiv bleibt. Erst wenn das Biosignale nicht mehr anliegt und die Person nicht mehr ruhig steht, wird der erhöhte Widerstand gegen eine Flexionsbewegung wieder verringert. Steht die Person jedoch wieder ruhig, kann die Ansteuerung durch das Biosignal beendet werden, um ein entspanntes Stehen zu ermöglichen. Dadurch wird die Person sowohl kognitiv als auch muskulär entlastet. Es ist ebenfalls möglich, dass durch die Ansteuerung mittels des Biosignals die Stehfunktion aktiviert wird oder aktiviert bleibt, obwohl in dieser Position die Aktivierung auf der Grundlage der übrigen Sensordaten normalerweise nicht möglich wäre. Ist beispielsweise eine Bedingung, dass ein Unterschenkelteil senkrecht oder in Gehrichtung nach vorne geneigt sein muss, bevor eine Stehfunktion mit einem erhöhten Flexionswiderstand eingeschaltet wird, wäre dies ein Hinderungsgrund, die Stellung in der rechten Darstellung der Figur 5 mit einer Stehfunktion zu versehen. Mit dem beanspruchten Verfahren ist es möglich, über das Biosignal die übrigen Bedingungen zu überstimmen und aufgrund einer höheren Priorisierung durch das Biosignal den Widerstand gegen eine Flexion zu erhöhen.

Eine weitere Situation ist in der Figur 6 dargestellt, bei der eine seitliche Bewegung innerhalb der Frontalebene stattfindet. In dem dargestellten Ausführungsbeispiel führt die Person einen Schritt nach links aus, während die versorgte rechte Seite stehen bleibt. Herkömmliche Steuerungen reagieren nicht auf Bewegungen innerhalb der Frontalebene, sodass über ein Biosignal auch bei solchen Situationen eine Stehfunktion mit einem erhöhten Flexionswiderstand eingestellt werden kann. Eine Erhöhung eines Flexionswiderstandes bei einer seitlichen Bewegung durch ein Biosignal ist zum Beispiel bei einem dynamischen Richtungswechsel von einer Vorwärtsbewegung zu einer kombinierten Vorwärts- und Seitwärtsbewegung sinnvoll, um sich von der versorgten Seite abstoßen zu können. In der terminalen Standphase kann trotz Ansteuerung über das Biosignal auf Basis der anderen Sensoren eine Reduktion des Flexionswiderstandes des Kniegelenks erfolgen, um eine Schwungphase einzuleiten oder ein Einbeugen für die Schwungphase zu unterstützen.

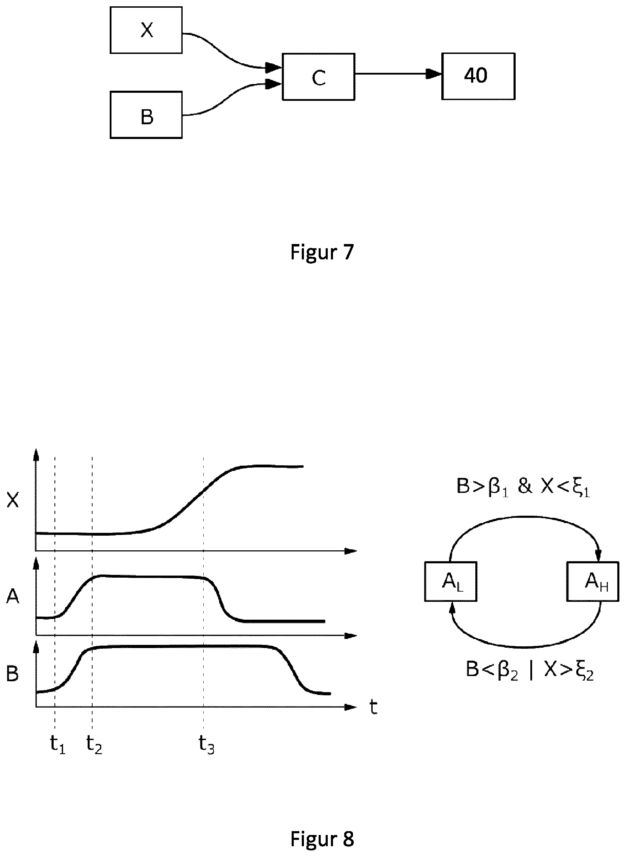

In der Figur 7 ist das grundsätzliche Funktionsschema der Steuerung dargestellt. Biosignale B und weitere Informationen X, die von Sensoren oder Datenquellen bereitgestellt werden, wie Sensorsignale über Zustandsgrößen, interne Zustände oder externe Daten, werden einer Steuerung C, die in einer Steuerungseinrichtung abgelegt und untergebracht ist, verarbeitet. Die Steuerung C der Steuerungseinrichtung steuert dann den Aktuator 40 an. Die konventionellen Informationen X sind typischerweise kinematische Größen wie Kniewinkel, Segmentwinkel, Ranges of Motion, Abstände, Längen und Orientierungen einer Beinsehne, Geschwindigkeiten, Beschleunigungen und dergleichen und kinetische Größen wie Kräfte, Momente, Hebelarme, Kraftangriffspunkte und dergleichen. Weiterhin können externe Informationen X von Einstelleinrichtung, Apps, anderen Prothesenkomponenten oder einer Daten-Cloud kommen. Die Biosignale B sind beispielsweise Daten hinsichtlich der Muskelanspannung, die über elektromyografische Sensoren, Ultraschall, Magnete, Drucksensoren, direkte Nervensensoren und dergleichen abgeleitet oder erfasst werden. Die Steuerung C in der Steuerungseinrichtung erzeugt eine Eingangsgröße für den Aktuator 40 und damit eine Stellgröße, zum Beispiel hinsichtlich der Position des Gelenks, der Position eines Ventils, das Öffnen oder das Schließen eines Ventils, über Geschwindigkeiten, Momente, Strom, Steifigkeiten, Dämpfungen, Impedanz und dergleichen.

In der Figur 8 ist eine Ausführungsform einer Steuerung für eine Erhöhung des Flexionswiderstandes schematisch dargestellt. Die Eingangsgröße X für Sensorwerte der Sensoren 50 gemäß der Figur 1 sind in der oberen Gruppe dargestellt, die Aktivierung oder Deaktivierung des Aktuators 40 ist in der mittleren Kurve mit dem Aktivierungssignal A über der Zeit dargestellt, das Biosignal B über der Zeit ist in der unteren Kurve eingezeichnet. In der rechten Darstellung der Figur 8 sind zwei der Aktuatorenzustände und die Bedingungen dafür eingezeichnet. Der erste Zustand AL bezeichnet einen Zustand, in dem der Aktuator 40 ein normales Widerstandsverhalten zeigt, also einen niedrigen Flexionswiderstand einnimmt bzw. offen ist. Der Aktuator Zustand AH entspricht dem Zustand mit einem erhöhten Widerstand, gegebenenfalls einen gesperrten Aktuator, der eine Flexion verhindert. Für den Fall, dass das Biosignal B größer als ein Grenzwert β1 ist und zusätzlich für die Sensorwerte oder konventionellen Größen X Werte vorliegen, die innerhalb eines Bereiches liegen, der normalerweise zu einer Aktivierung einer Stehfunktion führen würde, was durch den Ausdruck < ξ symbolisiert wird, wird von einem Zustand AL mit einem niedrigen Flexionswiderstand in einen Zustand AH mit einem hohen Flexionswiderstand umgeschaltet. Die Steuerung wird also nur dann aktiviert, wenn sowohl die Bedingung oder die Bedingungen für die Sensorwerte X der Zustandssensoren 50 als auch die Bedingungen der Biosignale B über die Biosignalsensoren 60 gemeinsam erfüllt sind. D. h., dass sich das System zum Beispiel hinreichend in Ruhe befindet und belastet ist und darüber hinaus eine Ansteuerung durch das Biosignal B vorhanden ist. Eine Deaktivierung, also eine Umschaltung von dem Zustand AH auf den Zustand AL, erfolgt dann, wenn einer der beiden Bedingungen nicht erfüllt ist. Wenn also kein Biosignal B in einer hinreichenden Größe oder Intensität vorhanden ist oder ein Grenzwert ξ für die Sensorwerte oder Zustandsdaten X überschritten ist, wird der Flexionswiderstand wieder verringert. Eine solche Umsetzung kann beispielsweise sinnvoll sein, wenn ein versehentliches Anheben oder eine Beibehaltung des Widerstandes besonders unerwünscht ist, zum Beispiel in einem Sondermodus wie dem Radfahren. In den Kurven ausgedrückt befindet sich die orthopädietechnische Gelenkeinrichtung zum Zeitpunkt t1 in Ruhe, ein Biosignal B ist nicht vorhanden. Der Widerstand des Aktuators 40 ist dementsprechend gering, wie es in der Kurve A gezeigt ist. Zwischen den Zeitpunkten t1 und t2 wird das Biosignal B angehoben, beispielsweise durch die Anspannung des Oberschenkelmuskels. Dementsprechend steigt auch der Widerstand A, der durch den Aktuator 40 bereitgestellt wird, wobei die Anhebung oder Erhöhung des Widerstandes proportional zu dem Muskelsignal erfolgen kann. Zu dem Zeitpunkt t3 findet eine ausreichende Veränderung des Zustandes der orthopädietechnischen Gelenkeinrichtung statt, was durch den Anstieg der Werte X angedeutet ist. Dementsprechend wird der Widerstand durch den Aktuator 40 verringert, obwohl das Biosignal B weiterhin anliegt.

In der Ausführungsform gemäß der Figur 8 wird das Biosignal B von den Sensorwerten X überstimmt oder overruled.

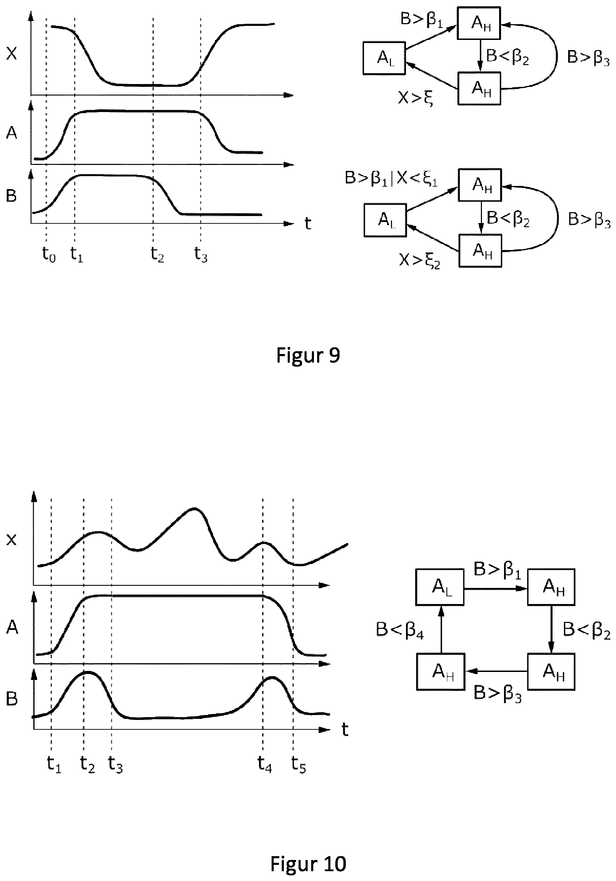

In der Figur 9 ist eine Variante der Steuerung dargestellt, bei der eine Deaktivierung, also eine Verringerung des Flexionswiderstandes, nur dann erfolgen, wenn keine Ansteuerung durch das Biosignal B vorhanden ist und auch die anderen Bedingungen durch die Zustandsdaten X der Gelenkeinrichtung nicht mehr erfüllt sind. Die Aktivierung erfolgt gemäß der Darstellung oben rechts in der Figur 9 nur in Abhängigkeit des Biosignals B, in der Darstellung unten rechts erfolgt die Aktivierung des Aktuators 40 dann, wenn entweder das Biosignal B ausreichend ist oder die Sensorwerte X für Zustandsdaten der orthopädietechnischen Gelenkeinrichtung in einer Größenordnung liegen, bei der eine Aktivierung der Stehfunktion und eine Erhöhung des Flexionswiderstandes erfolgen würde.

Zu dem Zeitpunkt t0 befindet sich die orthopädietechnische Gelenkeinrichtung in Gestalt einer Beinorthese oder eines Prothesenbeines in einer Vorwärtsbewegung und es erfolgt keine Ansteuerung durch das Biosignal B. Zwischen den Zeitpunkten t0 und t1 wird die Ansteuerung A durch das Biosignal B angehoben. Obwohl das Bein weiter nach vorne rotiert, was normalerweise zu keiner Aktivierung einer Stehfunktion aufgrund der Sensorwerte der Zustandsdaten X führen würde, wird der Widerstand über die Aktivierung des Aktuators 40 gemäß Kurve A erhöht. Durch die Anhebung des Flexionswiderstandes wird die Bewegung zwischen dem Oberteil und dem Unterteil verringert und gestoppt, und die Bewegung der orthopädietechnischen Einrichtung wird abgebremst, was sich an dem Abfall der Sensorwerte der Zustandsdaten X ablesen lässt. Das Biosignal B wird bei einer erfolgten Bremsung und Verriegelung des Gelenkes reduziert, beispielsweise indem der Muskel entspannt wird, sodass sich ab dem Zeitpunkt t2 die Intensität des Biosignals B verringert. Der erhöhte Flexionswiderstand bleibt bestehen, was durch den konstanten angehobenen Verlauf der Kurve A zum Ausdruck gebracht wird. Die Person kann entspannt stehen. Wird die Gelenkeinrichtung dann erneut bewegt, was sich an den ansteigenden Werten der X-Kurve ablesen lässt, wird der Widerstand gegen eine Flexion verringert, was die fallende Kurve A anzeigt.

In der oberen rechten Darstellung sind die Bedingungen für eine Umschaltung von dem Zustand AL auf den Zustand AH dargestellt, nämlich wenn das Biosignal B ausreichend groß ist und eine ausreichende Qualität hat, um eine Ansteuerung des Aktuators mit einer Umschaltung auf einen erhöhten Widerstand auszuführen. Wird auch das Biosignal B dann verringert oder fällt es weg, ist also das Biosignal B < β2, bleibt der Zustand des erhöhten Flexionswiderstandes bestehen. Gleiches geschieht, wenn erneut ein Biosignal B vorliegt, das größer als β3 ist, sodass eine ausreichende Signalqualität für eine Ansteuerung vorliegt. Erst wenn eine erneute Bewegung der orthopädietechnischen Gelenkeinrichtung ohne gleichzeitige Ansteuerung über das Biosignal stattfindet, also die Zustandswerte X > ξi sind, also außerhalb eines Bereiches liegen, der normalerweise zu einer Aktivierung einer Stehfunktion mit einem erhöhten Flexionswiderstand führen würde, wird der Flexionswiderstand verringert und der Zustand AL wird eingenommen. In der unteren rechten Darstellung wird eine Erhöhung des Flexionswiderstandes ausgeführt, wenn eine der beiden Bedingungen erfüllt sind, also entweder das Biosignal B größer als ein Grenzwert β ist, der eine Ansteuerung des Aktuators rechtfertigen würde oder wenn die Sensorwerte für die Zustandsgrößen innerhalb eines Bereiches liegen, der normalerweise zu einer Aktivierung der Stehfunktion und einer Erhöhung des Flexionswiderstandes führen würden. Eine Entriegelung bzw. Verringerung des Flexionswiderstandes findet erst dann statt, wenn die Sensorwerte für die Zustandsdaten X für die Gelenkeinrichtung außerhalb eines Bereiches liegen, der normalerweise zu einer Erhöhung des Flexionswiderstandes führen würde, beispielsweise wenn die gesamte Prothese angehoben und verschwenkt wird und nicht gleichzeitig eine Ansteuerung über das Biosignal stattfindet.

In der Figur 10 ist die Steuerung für einen Sondermodus dargestellt. Bei dem Sondermodus kann es sich beispielsweise um eine Sonderfunktion für das Fahrradfahren handeln, bei dem periodisch wiederkehrende Bewegungen ausgeführt werden. Innerhalb eines solchen Modus ist es dann möglich, dass nur durch das Biosignal B eine Erhöhung des Widerstandes erfolgt. Zu einem Zeitpunkt t1 wird das Biosignal B bis zu einem Zeitpunkt t2 erhöht, beispielsweise durch eine ansteigende Muskelkontraktion. Das Biosignal B bleibt auf einem höheren Niveau als zum Zeitpunkt t1. Gemeinsam mit der Erhöhung des Biosignals B findet eine Aktivierung des Aktuators mit einer Erhöhung des Flexionswiderstandes statt, was in der mittleren Kurve A gezeigt wird. Auch nach der Reduzierung und Beibehaltung der Muskelspannung auf einem erhöhten Niveau oberhalb eines Schwellwertes ab dem Zeitpunkt t2 bis zum Zeitpunkt t4 bleibt der Flexionswiderstand auf einem erhöhten Niveau. Die Erhöhung des Widerstandes kann auch durch die Qualität des Biosignals B ausgelöst werden, beispielsweise durch einen deutlichen Impuls oder über eine Zeitdauer der Aufrechterhaltung des Biosignals B. Die Bedingungen für die Qualität des Biosignals B dienen dazu, eine versehentliche Erhöhung des Flexionswiderstandes zu verhindern. Erst wenn erneut ein Biosignal B zu einem Zeitpunkt t4 oberhalb eines Niveaus auftritt und gegebenenfalls wieder abfällt, wird der Widerstand reduziert. Dementsprechend verändern sich auch die Bewegungen, was sich durch die Werte der Zustandsdaten X erkennen lässt. Diese Daten können dazu verwendet werden, eine andere Steuerungsstrategie oder einen anderen Steuerungsmodus zu aktivieren. Beispielsweise können beim Fahrradfahren diese Umschaltungen genutzt werden, um aufzustehen und auf den Pedalen zu stehen. Alleine die Pedalbewegungen oder Erschütterungen sollen nicht ausschlaggebend für das Aktivieren oder Deaktivieren des erhöhten Widerstandes sein.

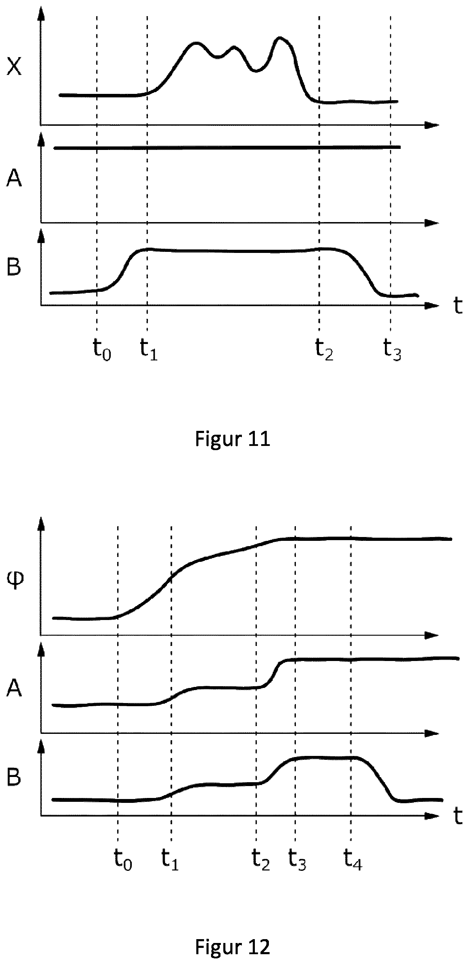

In der Figur 11 ist eine Variante der Steuerung gemäß der Figur 9 gezeigt, bei der die verriegelte Stellung oder der erhöhte Flexionswiderstand durch den hohen Werte der Kurve A angedeutet ist. Es erfolgt keine Ansteuerung durch das Biosignal B. Die die orthopädietechnische Gelenkeinrichtung nutzende Person steht beispielsweise entspannt in gebeugter Stellung, durch das Biosignal B kann nun eine Situation antizipiert werden, bei der der Widerstand einer herkömmlichen Steuerung reduziert werden würde, beispielsweise wenn die Zustandsdaten X eine Entriegelung oder Verringerung des Flexionswiderstandes zur Folge haben würde. Erfolgt bei einem bereits erhöhten Widerstand zum Zeitpunkt t0 eine Ansteuerung über das Biosignal B und wird erst danach eine Bewegung der orthopädietechnischen Gelenkeinrichtung ausgeführt, was durch die ansteigende und abfallende Kurve in dem Zeitraum zwischen der t1 und t2 der oberen Kurve X dargestellt ist, bleibt der Widerstand während der Bewegung in diesem Zeitraum hoch, was durch den konstanten Verlauf der Kurve A ausgedrückt wird. Wenn die Bewegung zum Zeitpunkt t2 beendet wird, kann auch das Biosignal B reduziert werden, was in dem Zeitraum zwischen Zeitpunkt t2 und t3 stattfindet. Auch die Bedingungen für eine Verringerung des Flexionswiderstandes auf der Grundlage der Sensorwerte der Zustandsdaten X befinden sich ab dem Zeitpunkt t2 in einem Bereich, in dem keine Entriegelung stattfinden würde, sodass die verriegelte Stellung oder die Stehfunktion bei einer orthopädietechnischen Gelenkeinrichtung der unteren Extremität weiterhin vorhanden ist. Der Flexionswiderstand bleibt weiterhin hoch, eine Entriegelung findet nicht statt.

In der Figur 12 ist eine weitere Steuerungsvariante gezeigt, bei der eine Beugebewegung über den Kniewinkel φk dargestellt wird. Der Kniewinkel φk ist in dem oberen Diagramm dargestellt. Zu Beginn der Steuerung liegt ein normales oder geringes Widerstandsverhalten für den Aktuator A vor. Eine Biosignal B liegt nicht an, das Kniegelenk befindet sich in einer hinreichend gestreckten Position. Wird die orthopädietechnische Gelenkeinrichtung, beispielsweise Gestalt einer Prothese wie in der Figur 3, auf die nächste Stufe nach unten gesetzt und beugt das künstliche Kniegelenk bei Belastung kontrolliert ein, was in dem Zeitraum zwischen t0 und t1 geschieht, wird zur Verringerung der Einbeugung ab dem Zeitpunkt t1 ein erstes Biosignal B auf einem erhöhten Niveau, beispielsweise eine mittelstarke Ansteuerung der Muskulatur, der Steuerung zugeführt, um einen mittleren Widerstand zu erreichen. Dieser Widerstand wird ab dem Zeitpunkt t1 aufgebaut und bis zum Zeitpunkt t2 beibehalten. Dies ermöglicht eine kontrollierte Verlangsamung der Beugebewegung, ohne diese abrupt zu beenden. Zu dem Zeitpunkt t2 soll die Bewegung gestoppt werden. Dazu wird das Biosignal B in dem Zeitraum zwischen den Zeitpunkten t2 und t3 erhöht, beispielsweise durch eine maximale Kontraktion des entsprechenden Muskels. Dadurch wird der Widerstand A, der durch den Aktuator bereitgestellt wird, maximal erhöht und die Veränderung des Kniewinkels φk wird verlangsamt, sodass die Bewegung zum Zeitpunkt t3 zum Stillstand kommt. Das Biosignal B kann zu dem Zeitpunkt t4, wenn die Kniebeugung vollständig zum Stillstand gekommen ist und eine Stehfunktion installiert ist, beendet werden, d. h., dass die Muskelspannung oder Muskelansteuerung verringert werden kann. Der Widerstand durch den Aktuator bleibt trotzdem hoch, bis ein entsprechendes Aufhebungssignal der Steuerung zugeführt wird.

Ergänzend oder alternativ zu einer Steuerung über einfache Schwellwerte, können kontinuierliche Übergänge zwischen den Widerständen realisiert werden. Kriterien können zum Beispiel teilweise erfüllt sein und zu einer teilweisen Erhöhung oder Verringerung des Widerstandes führen.

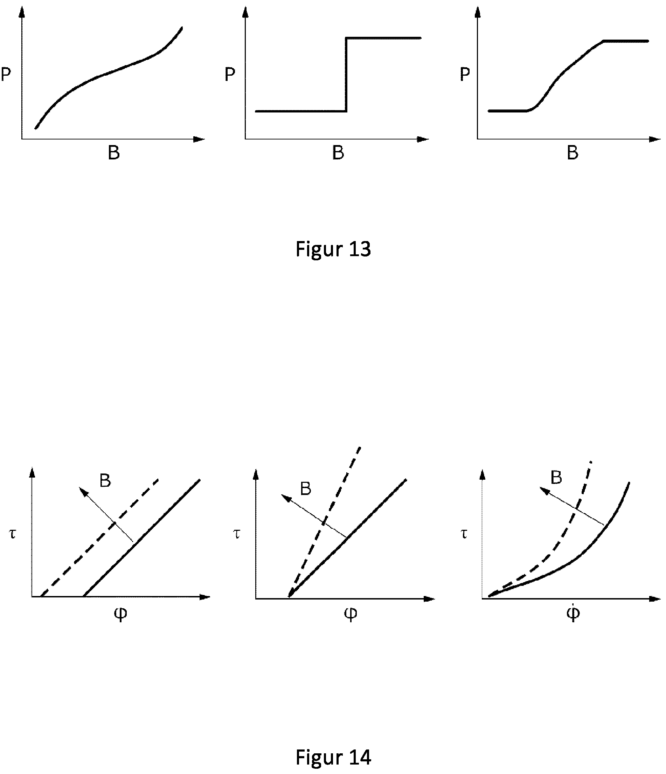

In der Figur 13 sind unterschiedliche Zusammenhänge zwischen dem Biosignal B und dem Widerstandsparameter P dargestellt. Durch die Ansteuerung über das Biosignal B wird das Widerstandsverhalten des Aktuators erhöht, wobei unterschiedliche Parameter P des Widerstandsverhaltens beeinflusst werden können. Dies wird in der Figur 14 näher erläutert werden. Die Art der Beeinflussung kann kontinuierlich bzw. proportional erfolgen, d. h., dass bei einer erhöhten Aktivierung der Muskulatur oder des Biosignals. B ein vergrößerter Widerstand bereitgestellt wird, was in der linken Darstellung der Figur 13 gezeigt ist. Der Zusammenhang zwischen dem Biosignal B und dem Widerstandsparameter P muss nicht linear sein. Alternativ kann die Anpassung binär erfolgen, d. h., dass eine festgelegte Erhöhung des Widerstandes ab einem bestimmten Schwellwert oder mehreren bestimmten Schwellwerten des Biosignals B ausgeführt wird. Dies ist in der mittleren Darstellung der Figur 13 dargestellt. Ebenfalls kann die Anpassung der Widerstandsparameter P Sättigungsbereiche aufweisen, sodass eine weitere Erhöhung der Ansteuerung durch das Biosignal B zu keiner weiteren Erhöhung des Widerstandes führt. Dies ist in der rechten Darstellung der Figur 13 dargestellt. Die unterschiedlichen Arten der Anpassung können auch miteinander kombiniert werden, ebenso können die unterschiedlichen Arten der Anpassung auch in unterschiedlichen Situationen unterschiedlich angewendet werden, beispielsweise kann eine binäre Umgestaltung beim Fahrradfahren erfolgen, während eine proportionale Anpassung beim Stehen erfolgt.

In der Figur 14 sind unterschiedliche Arten der Widerstandserhöhung an den Beispielen eines federnden und eines dämpfenden Verhaltensdargestellt, wobei ein Widerstandsmoment τ über dem Kniewinkel φi aufgetragen wird. Das Widerstandsmoment τ wird gegen eine Bewegung oder mit einer Bewegung aufgebracht, um eine Beeinflussung der Verschwenkbewegung zu bewirken. Bei einem federnden Verhalten kann eine Gleichgewichtsposition durch das Biosignal B verändert werden. Bei der Erzeugung eines das Knie streckenden Momentes, was durch das Einbeugen gegen ein Federverhalten erreicht wird, kann bei einer Ansteuerung über das Biosignal B die Gleichgewichtsposition der Feder zu einem kleineren Kniewinkel hin verschoben werden. Bei dem gleichen Kniewinkel φi liegt dann ein höheres Kniestreckmoment an. Ebenfalls kann die Federsteifigkeit verändert werden, was in der mittleren Darstellung der Figur 14 dargestellt ist. Bei einem dämpfenden Verhalten kann der Dämpfungskoeffizient mit der Ansteuerung über das Biosignal B erhöht werden. Bei nichtlinearen Charakteristiken sind entsprechend mehrere Koeffizienten zu verändern bzw. wird die entsprechende Kennlinie zu höheren Momenten hin verschoben. Die Erhöhung und/oder Verringerung eines Widerstandes kann damit auch die Erhöhung und/oder Verringerung einer Steifigkeit, eines Gleichgewichtspunktes, einer Dämpfung und/oder dergleichen sein. Es können auch anderer Parameter derartiger Charakteristiken im Sinne einer Widerstandsveränderung angepasst werden, zum Beispiel die Progressivität. Auch können mehrere Charakteristiken kombiniert werden, zum Beispiel ein federndes und ein dämpfendes Verhalten. Alternativ oder ergänzend zum Kniewinkel kann es sich bei φ um einen anderen Verschwenkungsfreiheitsgrad der orthopädietechnischen Einrichtung handeln, dem ein Widerstand entgegengebracht wird oder dessen Verschwenkbewegung durch einen Aktuator beeinflusst wird. Alternativ kann es sich aber auch um einen anderen, über die Sensoren erfassten Zustand der orthopädietechnischen Einrichtung handeln, wie den Absolutwinkel des Unter- oder Oberteils oder die Belastung. Durch das Biosignal kann natürlich auch direkt das Moment, bzw. die vom Aktuator aufgebracht Kraft, entgegen oder mit der Verschwenkung beeinflusst werden, insbesondere in einem proportionalen Zusammenhang, was einer Verringerung und/oder Erhöhung eines Widerstandes entspricht.

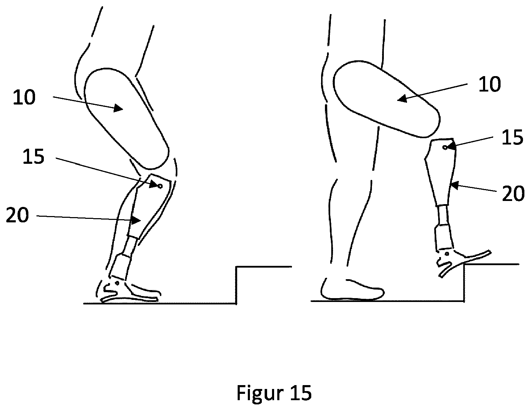

In der Figur 15 ist eine weitere Nutzungssituation einer orthopädietechnischen Gelenkeinrichtung dargestellt, nämlich das Repositionieren des versorgten Beines. In der links dargestellten Ausgangsstellung befindet sich die nutzende Person in leicht gebeugter Haltung. Eine Stehfunktion der orthopädietechnischen Einrichtung ist aktiv und stellt entsprechende Widerstände gegen eine Knieflexion sowie gegen eine Dorsalflexion zur Verfügung. Für die Repositionierung wird die versorgte Seite entlastet, das Bein nach vorne gesetzt, in diesem Fall auf eine Absatzkante, wie in der rechten Abbildung dargestellt, und dann erneut belastet. Bei einer Entlastung oder Verschwenkung würde eine gewöhnliche Stehfunktion deaktiviert und die Widerstände in Knie oder Knöchel reduziert werden. Eine Position wie rechts dargestellt einzunehmen und die Prothese zu belasten, wäre nur sehr schwer möglich, da es sich um eine sehr instabile Position handelt und sowohl Kniegelenk als auch Fußgelenk ein stark beugendes Moment erfahren würden. Wird jedoch ein Biosignal über die Biosignalsensoren, z.B. Ableiterelektroden der Steuerungseinrichtung übermittelt, ist es möglich, die Stehfunktion willentlich auch während der Entlastung und Repositionierung, in diesem Fall im Wesentlichen eine Verschwenkung um die Hüfte, aktiv zu halten. Nach dem Repositionieren und Belasten kann aus dieser quasistatischen Situation die Ansteuerung über das Biosignal wieder wegfallen, wobei die Stehfunktion trotzdem aktiv bleibt. Dies ist möglich, weil die Reduktion der Widerstände auf Basis von Sensordaten, insbesondere Sensordaten über den Zustand der orthopädietechnischen Einrichtung, erfolgt. Im dargestellten Fall der Repositionierung ist es vorteilhaft, nicht nur den Widerstand gegen eine Flexion, sondern auch den Widerstand gegen eine Extension im Knie und/oder gegen eine Plantarflexion im Knöchelgelenk auf Basis des Biosignals zu erhöhen, ggf. bis hin zu einer Sperre. Bei Wegfall des Biosignals nach dem Repositionieren kann der Widerstand gegen eine Extension und/oder eine Plantarflexion wieder reduziert werden.

## Patentansprüche

1. Verfahren zur Steuerung einer orthopädietechnischen Gelenkeinrichtung mit einem Oberteil (10) und einem Unterteil (20), die gelenkig aneinander um eine Schwenkachse (15) verschwenkbar gelagert sind, mit einem Aktuator (30), der mit dem Oberteil (10) und dem Unterteil (20) gekoppelt ist und eine Verschwenkbewegung des Oberteils (10) relativ zu dem Unterteil (20) beeinflusst, wobei der Aktuator (30) mit einer Steuerungseinrichtung (40) gekoppelt ist, die mit zumindest einem Sensor (50) zur Erfassung von Zustandsdaten der Gelenkeinrichtung gekoppelt ist und auf der Grundlage von Sensorwerten des zumindest einen Sensors (50) den Aktuator (30) aktiviert, deaktiviert oder moduliert, und mit zumindest einem Biosignalsensor (60), der eine Muskelaktivität oder eine Ansteuerung zumindest eines Muskels als Biosignal erfasst und der Steuerungseinrichtung (40) übermittelt, wobei auf der Grundlage des Biosignals oder der Biosignale der Aktuator (30) aktiviert, deaktiviert oder moduliert wird, wobei der Widerstand der Verschwenkbewegung durch den Aktuator (30) gegen eine Flexion auf der Grundlage zumindest eines Biosignals erhöht wird und anschließend der erhöhte Widerstand gegen eine Flexion auf der Grundlage von Sensorwerten verringert wird, **dadurch gekennzeichnet, dass** der erhöhte Flexionswiderstand unabhängig von einer Veränderung des Biosignals solange beibehalten wird, bis zumindest ein Sensorsignal aufgrund einer Bewegung, Belastung und/oder Zustandsänderung in einer definierten Größe von dem zumindest einem Sensor (50) detektiert wird.

2. Verfahren nach Anspruch 1, **dadurch gekennzeichnet, dass** der erhöhte Flexionswiderstand nur auf der Grundlage von Sensorwerten oder auf Grundlage einer Kombination aus Sensorwerten und zumindest einem Biosignal verringert wird.

3. Verfahren nach Anspruch 1 oder 2, **dadurch gekennzeichnet, dass** die Sensoren eine Bewegung des Oberteils (10) oder des Unterteils (20) absolut oder relativ zueinander detektieren.

4. Verfahren nach einem der voranstehenden Ansprüche, **dadurch gekennzeichnet, dass** das Biosignal unabhängig von der Position, Belastung und/oder Bewegung der Gelenkeinrichtung oder des Oberteils (10) oder des Unterteils (20) die Erhöhung des Flexionswiderstandes auslöst.

5. Verfahren nach einem der Ansprüche 1 bis 3, **dadurch gekennzeichnet, dass** das Biosignal die Erhöhung des Flexionswiderstandes nur auslöst, wenn keine oder eine unterhalb eines Grenzwertes liegende Position, Bewegung oder Zustandsänderung des Oberteils (10) und/oder Unterteils (20) durch den zumindest einen Sensor (50) und/oder eine Belastung oberhalb eines Grenzwertes, insbesondere eine Axialbelastung entgegen der Schwerkraftrichtung, des Oberteils (10) und/oder Unterteils (20) detektiert wird.

6. Verfahren nach einem der voranstehenden Ansprüche, **dadurch gekennzeichnet, dass** die Sensorwerte einen Schwellwert überschreiten oder unterschreiten müssen, bevor eine Verringerung des Flexionswiderstandes eingeleitet wird.

7. Verfahren nach einem der voranstehenden Ansprüche, **dadurch gekennzeichnet, dass** der Flexionswiderstand trotz Ansteuerung über das zumindest eine Biosignal auf Grundlage von zumindest einem Sensorsignal des zumindest einen Sensors (50) aufgrund einer Bewegung, Belastung und/oder Zustandsänderung in einer definierten Größe reduziert wird.

8. Verfahren nach einem der voranstehenden Ansprüche, **dadurch gekennzeichnet, dass** das Ausmaß der Erhöhung und/oder des Flexionswiderstandes auf Grundlage der Position, der Bewegung, der Belastung, der Zustandsänderung, dem Modus und/oder der Bewegungsphase verändert wird.

9. Verfahren nach einem der voranstehenden Ansprüche, **dadurch gekennzeichnet, dass** es sich bei dem zumindest einen Biosignal um ein myoelektrisches Signal handelt, das insbesondere durch implantierte oder oberflächlich angebrachte Elektroden erfasst wird.

10. Verfahren nach einem der voranstehenden Ansprüche, **dadurch gekennzeichnet, dass** der Flexionswiderstand bis zu einer Sperre erhöht wird.

11. Verfahren nach einem der voranstehenden Ansprüche, **dadurch gekennzeichnet, dass** der Flexionswiderstand auf der Grundlage von zumindest einem Sensorwert auf ein Niveau unterhalb des Ausgangswiderstandes vor Erhöhung des Flexionswiderstandes verringert wird, insbesondere in einer terminalen Standphase.

12. Verfahren nach einem der voranstehenden Ansprüche, **dadurch gekennzeichnet, dass** in zumindest einem Modus oder zumindest einer Bewegungsphase die Ansteuerung eines Muskels oder einer Muskelgruppe erfasst und für die Steuerung herangezogen wird.

13. Verfahren nach einem der voranstehenden Ansprüche, **dadurch gekennzeichnet, dass** in zumindest einem Modus oder zumindest einer Bewegungsphase die Kokontraktion von zumindest zwei Muskeln oder Muskelgruppen erfasst und für die Steuerung herangezogen wird.

14. Verfahren nach einem der voranstehenden Ansprüche, **dadurch gekennzeichnet, dass** der Flexionswiderstand kontinuierlich mit erhöhter Aktivierung der Muskulatur erhöht und/oder mit verringerter Aktivierung verringert wird und/oder der Flexionswiderstand digital über die Aktivierung der Muskulatur angesteuert wird.

15. Orthopädietechnische Gelenkeinrichtung mit einem Oberteil (10) und einem Unterteil (20), die gelenkig aneinander um eine Schwenkachse (15) verschwenkbar gelagert sind, mit einem Aktuator (30), der mit dem Oberteil (10) und dem Unterteil (20) gekoppelt ist und eine Verschwenkbewegung des Oberteils (10) relativ zu dem Unterteil (20) beeinflusst, wobei der Aktuator (30) mit einer Steuerungseinrichtung (40) gekoppelt ist, die mit zumindest einem Sensor (50) zur Erfassung von Zustandsdaten der Gelenkeinrichtung gekoppelt ist und auf der Grundlage von Sensorwerten des zumindest einen Sensors (50) den Aktuator (30) aktiviert, deaktiviert oder moduliert, und mit zumindest einem Biosignalsensor (60), der Biosignale erfasst und der Steuerungseinrichtung (40) übermittelt, wobei auf der Grundlage der Biosignale der Aktuator (30) aktiviert, deaktiviert oder moduliert wird, wobei die Steuerungseinrichtung (40) eingerichtet ist, die Beeinflussung durch den Aktuator (30) gegen eine Flexion auf der Grundlage eines Biosignals zu erhöhen und den erhöhten Flexionswiderstand auf der Grundlage von Sensorwerten zu verringern und **dadurch gekennzeichnet, dass** der erhöhte Flexionswiderstand unabhängig von einer Veränderung des Biosignals solange beibehalten wird, bis zumindest ein Sensorsignal aufgrund einer Bewegung, Belastung und/oder Zustandsänderung in einer definierten Größe von dem zumindest einem Sensor (50) detektiert wird.

16. Orthopädietechnischen Gelenkeinrichtung nach Anspruch 15, **dadurch gekennzeichnet, dass** sie als Gelenkeinrichtung der unteren Extremität, insbesondere Orthesenhüftgelenk, Prothesenhüftgelenk, Orthesenkniegelenk, Prothesenkniegelenk, Orthesenknöchelgelenk, Prothesenknöchelgelenk oder als Gelenkeinrichtung der oberen Extremität, insbesondere als künstliches Ellenbogengelenk oder künstliches Schultergelenk eine Orthese, Prothese oder eines Exoskelettes ausgebildet ist.

17. Orthopädietechnischen Gelenkeinrichtung nach Anspruch 15 oder 16, **dadurch gekennzeichnet, dass** der zumindest eine Sensor (50) an dem Oberteil (10) und/oder dem Unterteil (20) angeordnet ist.

## Claims

1. A method for controlling an orthopedic joint device comprising an upper part (10) and a lower part (20), which are mounted articulated to one another so that they can pivot about a pivot axis (15), comprising an actuator (30), which is coupled to the upper part (10) and to the lower part (20) and influences a pivoting movement of the upper part (10) relative to the lower part (20), the actuator (30) being coupled to a control device (40), which is coupled to at least one sensor (50) for recording state data of the joint device and activates, deactivates or modulates the actuator (30) on the basis of sensor values of the at least one sensor (50) and comprising at least one biosignal sensor (60), which records a muscle activity or a stimulation of at least one muscle as a biosignal and transmits it to the control device (40), the actuator (30) being activated, deactivated or modulated on the basis of the biosignal or the biosignals, wherein the resistance to the pivoting movement by the actuator (30) against a flexion is increased on the basis of at least one biosignal, and the increased resistance against a flexion is subsequently reduced on the basis of sensor values, **characterized in that** the increased flexion resistance is maintained, independently of a variation of the biosignal, until at least one sensor signal due to a movement, load and/or state change of a defined size is detected by the at least one sensor (50).

2. The method as claimed in claim 1, **characterized in that** the increased flexion resistance is reduced only on the basis of sensor values or on the basis of a combination of sensor values and at least one biosignal.

3. The method as claimed in claim 1 or 2, **characterized in that** the sensors detect a movement of the upper part (10) or of the lower part (20) absolutely or relative to one another.

4. The method as claimed in one of the preceding claims, **characterized in that** the biosignal triggers the increase of the flexion resistance independently of the position, load and/or movement of the joint device or of the upper part (10) or of the lower part (20).

5. The method as claimed in one of claims 1 to 3, **characterized in that** the biosignal triggers the increase of the flexion resistance only if no position, movement or state change of the upper part (10) and/or lower part (20), or one that lies below a limit value, is detected by the at least one sensor (50) and/or a load above a limit value, in particular an axial load against the direction of gravity, of the upper part (10) and/or lower part (20) is detected.

6. The method as claimed in one of the preceding claims, **characterized in that** the sensor values must exceed or fall below a threshold value before a reduction of the flexion resistance is initiated.

7. The method as claimed in one of the preceding claims, **characterized in that** the flexion resistance is reduced despite stimulation via the at least one biosignal on the basis of the at least one sensor signal of the at least one sensor (50) due to a movement, load and/or state change of a defined size.

8. The method as claimed in one of the preceding claims, **characterized in that** the extent of the increase and/or of the flexion resistance is varied on the basis of the position, the movement, the load, the state change, the mode and/or the movement phase.

9. The method as claimed in one of the preceding claims, **characterized in that** the at least one biosignal is a myoelectrical signal, which is recorded in particular by implanted electrodes or electrodes fitted on the surface.

10. The method as claimed in one of the preceding claims, **characterized in that** the flexion resistance is increased to the extent of blocking.

11. The method as claimed in one of the preceding claims, **characterized in that** the flexion resistance is reduced on the basis of at least one sensor value to a level below the initial resistance before increasing the flexion resistance, particularly in a terminal stance phase.

12. The method as claimed in one of the preceding claims, **characterized in that**, in at least one mode or at least one movement phase, the stimulation of a muscle or a muscle group is recorded and employed for the control.

13. The method as claimed in one of the preceding claims, **characterized in that**, in at least one mode or at least one movement phase, the cocontraction of at least two muscles or muscle groups is recorded and employed for the control.

14. The method as claimed in one of the preceding claims, **characterized in that** the flexion resistance is increased continuously with increased activation of the musculature and/or reduced with reduced activation and/or the flexion resistance is controlled digitally by the activation of the musculature.

15. An orthopedic joint device comprising an upper part (10) and a lower part (20), which are mounted articulated to one another so that they can pivot about a pivot axis (15), comprising an actuator (30), which is coupled to the upper part (10) and to the lower part (20) and influences a pivoting movement of the upper part (10) relative to the lower part (20), the actuator (30) being coupled to a control device (40), which is coupled to at least one sensor (50) for recording state data of the joint device and activates, deactivates or modulates the actuator (30) on the basis of sensor values of the at least one sensor (50) and comprising at least one biosignal sensor (60), which records biosignals and transmits them to the control device (40), the actuator (30) being activated, deactivated or modulated on the basis of the biosignals, wherein the control device (40) is adapted to increase the influencing by the actuator (30) against a flexion on the basis of a biosignal and to reduce the increased flexion resistance on the basis of sensor values, **characterized in that** the increased flexion resistance is maintained, independently of a variation of the biosignal, until at least one sensor signal due to a movement, load and/or state change of a defined size is detected by the at least one sensor (50).

16. The orthopedic joint device as claimed in claim 15, **characterized in that** it is formed as a joint device of the lower extremity, in particular an orthotic hip joint, prosthetic hip joint, orthotic knee joint, prosthetic knee joint, orthotic ankle joint, prosthetic ankle joint or as a joint device of the upper extremity, in particular as an artificial elbow joint or artificial shoulder joint an orthosis, prosthesis or of an exoskeleton.

17. The orthopedic joint device as claimed in claim 15 or 16, **characterized in that** the at least one sensor (50) is arranged on the upper part (10) and/or on the lower part (20).

## Revendications

1. Procédé de commande d'un dispositif d'articulation orthopédique comprenant une partie supérieure (10) et une partie inférieure (20) qui sont montées en articulation l'une sur l'autre de manière à pouvoir pivoter autour d'un axe de pivotement (15), comprenant un actionneur (30) qui est couplé à la partie supérieure (10) et à la partie inférieure (20) et qui influence un mouvement de pivotement de la partie supérieure (10) par rapport à la partie inférieure (20), l'actionneur (30) étant couplé à un dispositif de commande (40) qui est couplé à au moins un capteur (50) destiné à acquérir des données d'état du dispositif d'articulation et qui, sur la base des valeurs de mesure dudit au moins un capteur (50), active, désactive ou module l'actionneur (30), et comprenant au moins un capteur de signaux biologiques (60) qui détecte une activité musculaire ou une activation d'au moins un muscle en tant que signal biologique et le transmet au dispositif de commande (40), l'actionneur (30) étant activé, désactivé ou modulé sur la base du signal biologique ou des signaux biologiques, la résistance à la flexion du mouvement de pivotement exercée par l'actionneur (30) étant augmentée sur la base d'au moins un signal biologique, puis la résistance accrue à la flexion étant réduite sur la base de valeurs de capteur, **caractérisé en ce que** la résistance accrue à la flexion est maintenue indépendamment de toute variation du signal biologique jusqu'à ce qu'au moins un signal de capteur soit détecté par ledit au moins un capteur (50) en raison d'un mouvement, d'une sollicitation et/ou d'un changement d'état d'une amplitude définie.

2. Procédé selon la revendication 1,
**caractérisé en ce que** la résistance accrue à la flexion n'est réduite que sur la base de valeurs de capteur ou sur la base d'une combinaison de valeurs de capteur et d'au moins un signal biologique.

3. Procédé selon la revendication 1 ou 2,
**caractérisé en ce que** les capteurs détectent un mouvement de la partie supérieure (10) ou de la partie inférieure (20) de manière absolue ou relative l'une par rapport à l'autre.

4. Procédé selon l'une des revendications précédentes,
**caractérisé en ce que** le signal biologique déclenche l'augmentation de la résistance à la flexion indépendamment de la position, de la sollicitation et/ou du mouvement du dispositif d'articulation ou de la partie supérieure (10) ou de la partie inférieure (20).

5. Procédé selon l'une des revendications 1 à 3,
**caractérisé en ce que** le signal biologique ne déclenche l'augmentation de la résistance à la flexion que lorsqu'aucune position, aucun mouvement ou aucun changement d'état de la partie supérieure (10) et/ou de la partie inférieure (20) n'est détecté(e) par ledit au moins un capteur (50) ou bien lorsque la position, le mouvement ou le changement d'état est inférieur(e) à une valeur limite, ou lorsqu'une sollicitation est détectée qui est supérieure à une valeur limite, en particulier une sollicitation axiale opposée à la direction de la gravité, de la partie supérieure (10) et/ou de la partie inférieure (20).

6. Procédé selon l'une des revendications précédentes,
**caractérisé en ce que** les valeurs de capteur doivent être supérieures ou être inférieures à une valeur seuil avant qu'une réduction de la résistance à la flexion ne soit déclenchée.

7. Procédé selon l'une des revendications précédentes,
**caractérisé en ce que** la résistance à la flexion est réduite, malgré la commande par ledit au moins un signal biologique, sur la base d'au moins un signal de capteur provenant dudit au moins un capteur (50), en raison d'un mouvement, d'une sollicitation et/ou d'un changement d'état d'une amplitude définie.

8. Procédé selon l'une des revendications précédentes,
**caractérisé en ce que** l'ampleur de l'augmentation et/ou de la résistance à la flexion est modifiée sur la base de la position, du mouvement, de la sollicitation, du changement d'état, du mode et/ou de la phase de mouvement.

9. Procédé selon l'une des revendications précédentes,
**caractérisé en ce que** ledit au moins un signal biologique est un signal myoélectrique qui est notamment capté par des électrodes implantées ou placées en surface.

10. Procédé selon l'une des revendications précédentes,
**caractérisé en ce que** la résistance à la flexion est augmentée jusqu'à un seuil de blocage.

11. Procédé selon l'une des revendications précédentes,
**caractérisé en ce que** la résistance à la flexion est réduite, sur la base d'au moins une valeur de capteur, à un niveau inférieur à la résistance initiale avant l'augmentation de la résistance à la flexion, en particulier dans une phase terminale de la phase d'appui.

12. Procédé selon l'une des revendications précédentes,
**caractérisé en ce que**, dans au moins un mode ou dans au moins une phase de mouvement, l'activation d'un muscle ou d'un groupe de muscles est détectée et utilisée pour la commande.

13. Procédé selon l'une des revendications précédentes,
**caractérisé en ce que**, dans au moins un mode ou dans au moins une phase de mouvement, la co-contraction d'au moins deux muscles ou groupes de muscles est détectée et utilisée pour la commande.

14. Procédé selon l'une des revendications précédentes,
**caractérisé en ce que** la résistance à la flexion est augmentée en continu au fur et à mesure d'une augmentation de l'activation de la musculature et/ou est réduite en continu au fur et à mesure d'une réduction de l'activation, et/ou la résistance à la flexion est commandée numériquement via l'activation de la musculature.

15. Dispositif d'articulation orthopédique comprenant une partie supérieure (10) et une partie inférieure (20) qui sont montées en articulation l'une sur l'autre de manière à pouvoir pivoter autour d'un axe de pivotement (15), comprenant un actionneur (30) qui est couplé à la partie supérieure (10) et à la partie inférieure (20) et qui influence un mouvement de pivotement de la partie supérieure (10) par rapport à la partie inférieure (20), l'actionneur (30) étant couplé à un dispositif de commande (40) qui est couplé à au moins un capteur (50) destiné à acquérir des données d'état du dispositif d'articulation et qui, sur la base des valeurs de mesure dudit au moins un capteur (50), active, désactive ou module l'actionneur (30), et comprenant au moins un capteur de signaux biologiques (60) qui détecte des signaux biologiques et les transmet au dispositif de commande (40), l'actionneur (30) étant activé, désactivé ou modulé sur la base des signaux biologiques,
le dispositif de commande (40) étant conçu pour augmenter l'influence de l'actionneur (30) à l'encontre d'une flexion sur la base d'un signal biologique et pour réduire la résistance accrue à la flexion sur la base de valeurs de capteur,
**caractérisé en ce que** la résistance accrue à la flexion est maintenue indépendamment de toute variation du signal biologique jusqu'à ce qu'au moins un signal de capteur soit détecté par ledit au moins un capteur (50) en raison d'un mouvement, d'une sollicitation et/ou d'un changement d'état d'une amplitude définie.

16. Dispositif d'articulation orthopédique selon la revendication 15,
**caractérisé en ce qu'**il est utilisé en tant que dispositif d'articulation du membre inférieur, en particulier en tant qu'orthèse d'articulation de la hanche, prothèse d'articulation de la hanche, orthèse d'articulation du genou, prothèse d'articulation du genou, orthèse d'articulation de la cheville, prothèse d'articulation de la cheville, ou en tant que dispositif d'articulation du membre supérieur, en particulier en tant qu'articulation artificielle du coude ou articulation artificielle de l'épaule, orthèse, prothèse ou exosquelette.

17. Dispositif d'articulation orthopédique selon la revendication 15 ou 16,
**caractérisé en ce que** ledit au moins un capteur (50) est disposé sur la partie supérieure (10) et/ou sur la partie inférieure (20).
